# EUROPEAN PATENT APPLICATION

(11) **EP 3 839 043 A1**
(43) Date of publication of application: **23.06.2021**
(21) Application number: 19218874.6
(22) Date of filing: 20.12.2019
(51) Int. Cl.: C12N 5/00

(54) **SCREENING METHODS FOR COMPOUNDS MODULATING POLYAMINE TRANSPORT**

(71) Applicant: Katholieke Universiteit Leuven, 3000 Leuven (BE)
(72) Inventor: AGOSTINIS, Patrizia, 3010 Kessel Lo (BE); ANNAERT, Wim, 2550 Kontich (BE); BAEKELANDT, Veerle, 3001 Heverlee (BE); EGGERMONT, Jan, 3220 Holsbeek (BE); KAHLER, Jan Pascal, 3000 Leuven (BE); MARTIN, Shaun, 3390 Tielt-Winge (BE); SWINNEN, Johan, 3110 Rotselaar (BE); VAN DEN HAUTE, Chris, 3012 Wilsele (BE); VAN VEEN, Sarah, 3390 Tielt-Winge (BE); VANGHELUWE, Peter, 3018 Wijgmaal (BE); VERHELST, Steven, 3010 Kessel-Lo (BE); VANHUTTE, Roeland, 3000 Leuven (BE)

(57) **Abstract**

The invention relates to vitro methods of identifying compounds modulating (stimulating) polyamine transport into a cell comprising the step of:
a) providing a first eukaryotic cell culture wherein the endogenous P5B type ATPase gene is inactivated, wherein the cells comprise an inserted copy of a **wild type** or a **disease causing mutant** of the P5B type ATPase, and providing a second eukaryotic cell culture wherein the endogenous P5B type ATPase gene is inactivated, wherein the cells comprise an inserted copy of **an inactive** P5B type ATPase,
b) contacting the first and the second cell culture with a labelled polyamine and with a test compound,
c) measuring the uptake of the labelled polyamine by the first and the second cell culture,
d) determining the difference in uptake between the two cell cultures, wherein a difference in uptake between the first and the second culture, indicates that the test compound modulates polyamine transport.

## Description

### Field of the invention

The invention relates to P5B type ATPase such as PTP13A2

The invention relates to screening methods for detecting compounds modulating P5B type ATPase and compounds modulating polyamine transport.

### Background of the invention

Parkinson's disease (PD) is one of the most common neurodegenerative disorders that at the cellular level is marked by abnormal protein aggregation¹, oxidative stress, and lysosomal and mitochondrial dysfunction². While the discovery of over twenty PD-associated genes revealed central pathways in the mechanism underlying PD³, hitherto, the function of several PD genes remains unclear. Mutations in one of these genes, *ATP13A2,* were first identified as the underlying cause of Kufor-Rakeb syndrome (KRS), a rare early-onset form of parkinsonism with dementia⁴. Later, disease-causing mutations in *ATP13A2* were discovered in early-onset PD (EOPD)⁵, neuronal ceroid lipofuscinosis (NCL; lysosomal storage disorder)⁶, hereditary spastic paraplegia (HSP; progressive gait disorder)⁷ and juvenile-onset amyotrophic lateral sclerosis (motor neuron disease)⁸. ATP13A2 is a late endo-lysosomal orphan transporter that imparts neuroprotection. ATP13A2 expression is increased in surviving neurons of sporadic PD brains⁹, while at the cellular level, ATP13A2 offers protection against genetic and environmental risk factors of PD, ranging from α-synuclein¹⁰ to heavy metal¹¹ toxicity and mitochondrial complex I inhibition^{12,13}. Loss of ATP13A2 leads to compromised lysosomal function¹⁴ and mitochondrial fragmentation¹⁵, but the unknown lysosomal transport function of ATP13A2 remains a critical bottleneck for resolving its general neuroprotective role.

De La Hera et al. Biochem J 450, 47-53, (2013), suggest a role for polyamines on ATP13A2 activity. The experimental conditions used thereon suggest an incorrect direction of polyamine transport, making it impossible to design a reliable screening assay based on polyamine transport.

### Summary of the invention

ATP13A2 belongs to the family of P-type ATPases, which couple ATP hydrolysis to substrate transport while transiently forming a catalytic phospho-intermediate¹⁶. Five P-type subfamilies exist (P1-5), which transport heavy metals (P1), ions (P2), protons (P3; solely fungi and plants) or lipids (P4; flippases)¹⁷. ATP13A2 is one of five human isoforms (ATP13A1-5) that belong to the orphan P5 subfamily, which was identified by genome sequencing in eukaryotes over twenty years ago¹⁸. ATP13A2 is generally considered a heavy metal transporter^{10,19,20}, but calcium²¹, magnesium²², the polyamine spermidine (SPD)^{23,24} and lipids²⁵ have also been mentioned as putative substrates.
Here, we identified the polyamines spermine (SPM) and SPD as the preferred transport substrates of ATP13A2 and showed that disease-associated mutations hamper this polyamine transport function. Moreover, ATP13A2 removes polyamines from the lysosome, whereas dysfunctional ATP13A2 leads to lysosomal accumulation of polyamines resulting in disturbed lysosomal functionality. This represents a novel, polyamine-driven lysosome-dependent cell death pathway that may contribute to the onset of neurodegeneration.

The invention is summarised in the following statements:
1. An in vitro method of identifying compounds modulating (stimulating) polyamine transport into a cell comprising the step of:
   a) providing a first eukaryotic cell culture wherein the endogenous P5B type ATPase gene is inactivated, wherein the cells comprise an inserted copy of a **wild type** or a **disease causing mutant** of the P5B type ATPase, and
      providing a second eukaryotic cell culture wherein the endogenous P5B type ATPase gene is inactivated, wherein the cells comprise an inserted copy of **an inactive** P5B type ATPase,
   b) contacting the first and the second cell culture with a labelled polyamine and with a test compound,
   c) measuring the uptake of the labelled polyamine by the first and the second cell culture,
   d) determining the difference in uptake between the two cell cultures, wherein a difference in uptake between the first and the second culture, indicates that the test compound modulates polyamine transport.
2. The method according to statement 1, wherein the P5B type ATPase is ATP13A2.
3. The method according to statement 1 or 2, wherein the inactive ATPase has no autophosphorylation, dephosphorylation, ATPase and/or transport activity.
4. The method according to any one of statements , wherein the eukaryotic cells are mammalian cells.
5. The method according to any one of statements 1 to 4, for identifying compounds stimulating polyamine transport.
6. The method according to any one of statements 1 to 5, wherein the labelled polyamine is a fluorescently labelled polyamine.
7. The method according to statement 6, wherein the uptake of fluorescent polyamine is determined by FACS analysis of cells.
8. The method according to statement 6 or 7, wherein prior to the measurement in step c), extracellular fluorescent polyamine is washed away or quenched.
9. The method according to any one of statements 1 to 8, wherein the polyamine is putrescine, spermine or spermidine.
10. The method according to any one of statements 1 to 9, wherein the cell cultures are further subjected to oxidative stress.
11. Use of the method of any one of statements 1 to 10, for detection of components activating P5B type ATPase having neuroprotective properties.
12. Use of the method of any one of statements 1 to 10, for detection of components inhibiting P5B type ATPase having antitumor properties.
13. Use of the method of any one of statements 1 to 10, for detection of components activating P5B Type ATPase, having cardiovascular activity.
14. A eukaryotic, typically mammalian, cell line wherein the endogenous P5B type ATPase gene is inactivated, **characterised in that** the cell line comprises an inserted copy of a wild type or mutant P5B type ATPase.
15. The cell line according to statement 14, wherein the endogenous P5B type ATPase gene is inactivated or mutated by crispr/cas.
16. The cell line according to statement 14 or 15, wherein the inserted copy is delivered via a viral vector, such as an adenoviral vector, an AAV vector, or a lentiviral vector.
17. A kit comprising:
   a first eukaryotic (mammalian) cell line wherein the endogenous P5B type ATPase gene is inactivated, characterised in that the cell line comprises an inserted copy of a **wild type or disease causing mutant** P5B type ATPase, and
   a second eukaryotic (mammalian) cell line wherein the endogenous P5B type ATPase gene is inactivated, characterised in that the cell line comprises an inserted copy of a an inactivated P5B type ATPase.
18. The kit according to statement 17, wherein the first and the second cell line have comparable expression levels of said inserted ATPase.
19. The kit according to statement 17 or 18, wherein the first and the second cell line have comparable endocytosis activity.
20. The kit according to any one of statements 17 to 19, wherein an inactivated P5B type ATPase is a catalytic inactive mutant lacking autophosphorylation, dephosphorylation, ATPase or transport activity.
21. The kit according to any one of statements 17 to 20, wherein the catalytic inactive mutant is the D508N mutant, with reference to the numbering of ATP13A2 variant 2.
22. The kit according to any one of statements 17 to 21, wherein the cell line is a primary or immortalised brain cell line.
23. The kit according to any one of statements 17 to 22, wherein the cell line is a neuroblastoma cell line.
24. An in vitro method of screening compounds modulating P5B type ATPase activity comprising the steps of:
   a) providing a cell membrane fraction of cells expressing wild type P5B type ATPase or a disease causing mutant thereof in a buffered solution comprising:
      - polyamine as transported substrate
      - ATP
      - a solubilising or membrane permeabilizing agent
      - and a test compound
   b) measuring ATP hydrolysis (by e.g. luminescence by measuring the released ADP, or colorimetric by measuring inorganic phosphate production)
   c) comparing the measured ATP hydrolysis with the value of a control experiment without test compound, with vehicle control or with reference compounds that are modulators of P5B ATPase or polyamine transport activity.
   d) wherein a difference in ATP hydrolysis is indicative of a test compound modulation P5B type ATPase activity .
25. An in vitro method of screening compounds modulating recombinant and purified P5B ATPase activity comprising the steps of:
   a) Providing a buffered solution comprising solubilised purified P5B-ATPase such as ATP13A2
      - a polyamine
      - ATP
      - and a regulatory lipid or agent that targets the auto-regulatory domain of the P5B-ATPase
   b) adding a test compound,
   c) determining ATPase activity, and comparing the determined active with the activity of a control experiment without test compound, with vehicle control or with reference compounds that are modulators of P5B ATPase or polyamine transport activity.

### DETAILED DESCRIPTION

### Figure legends

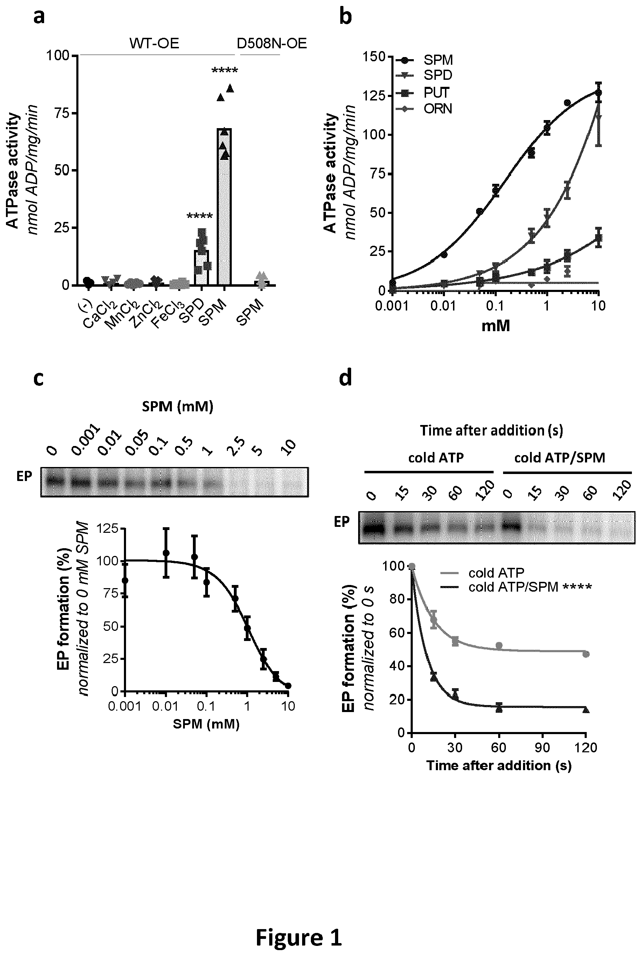

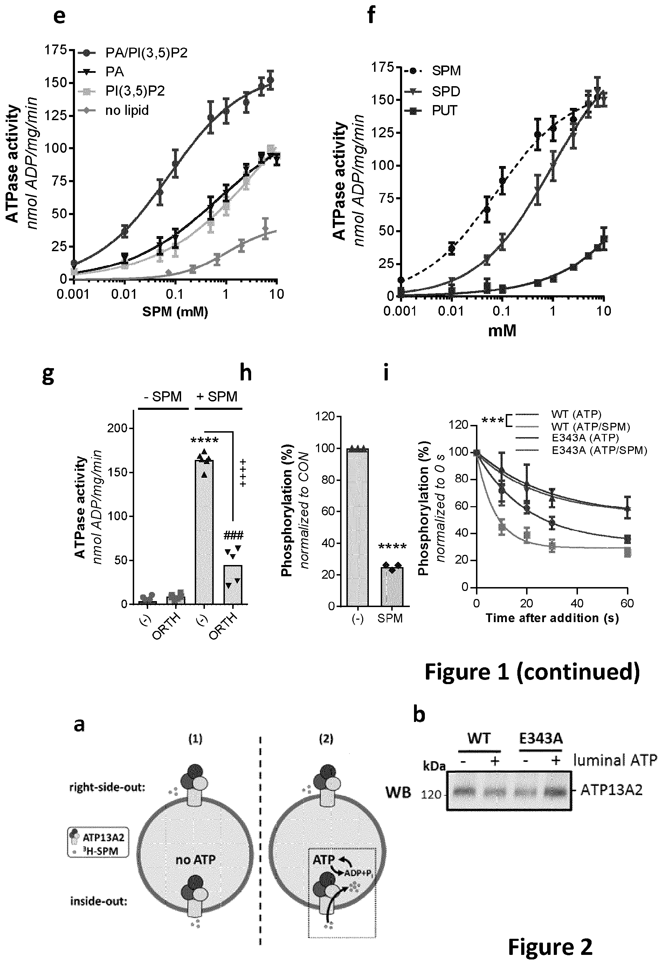

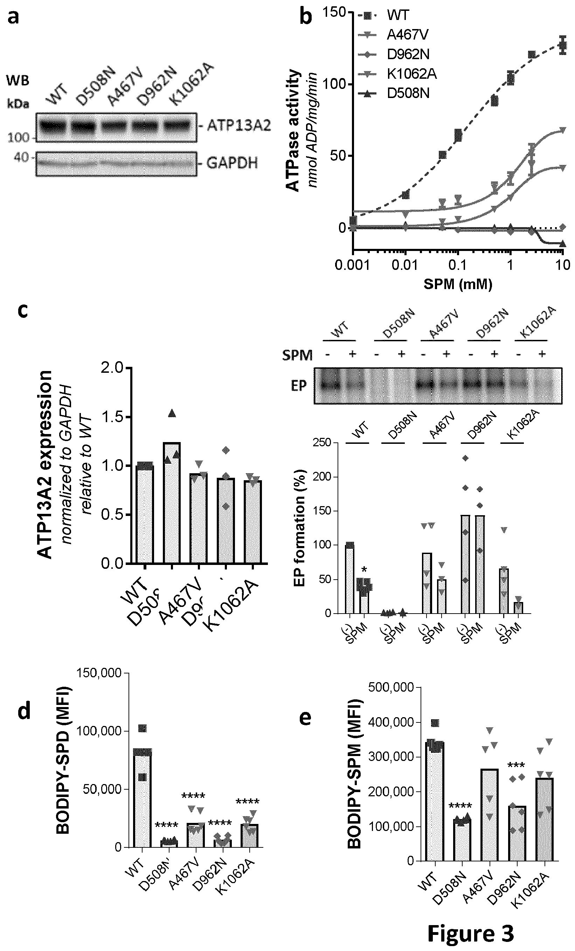

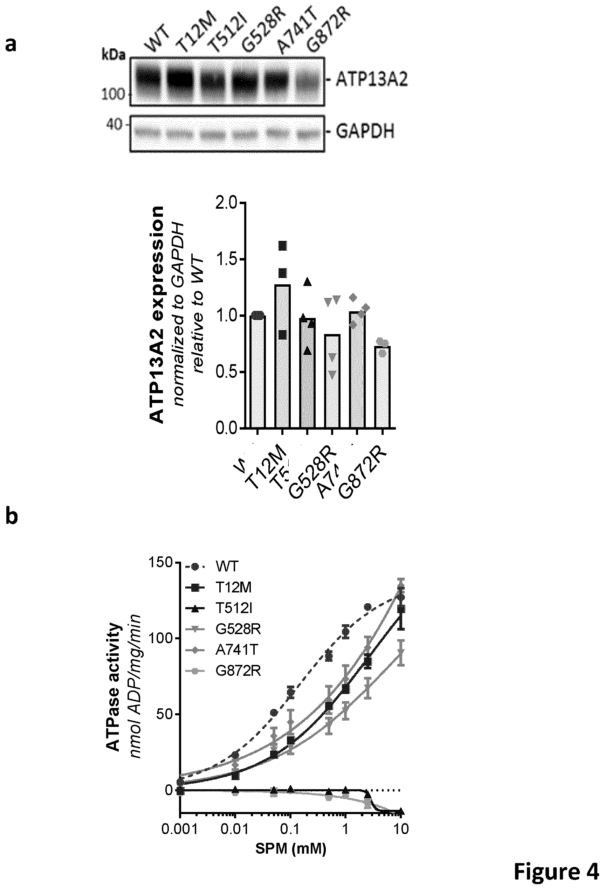

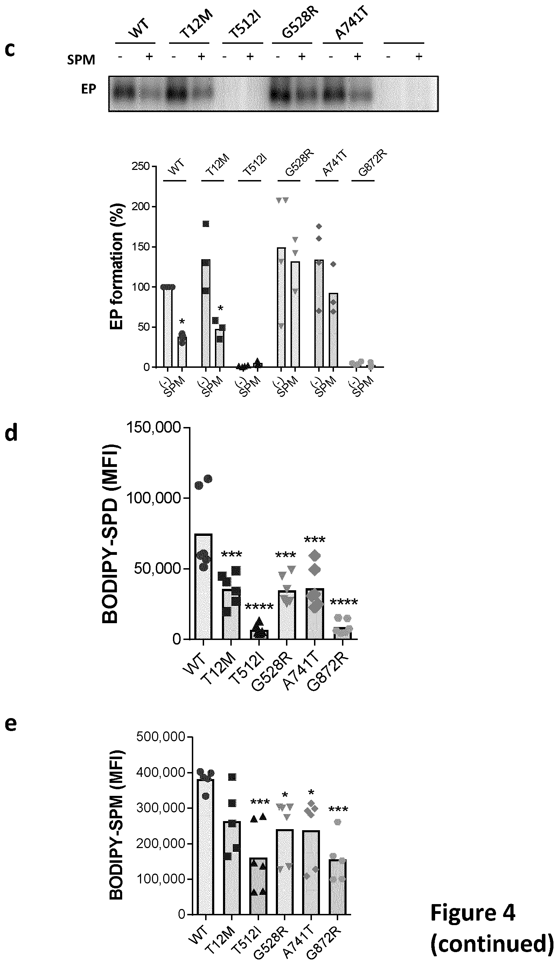

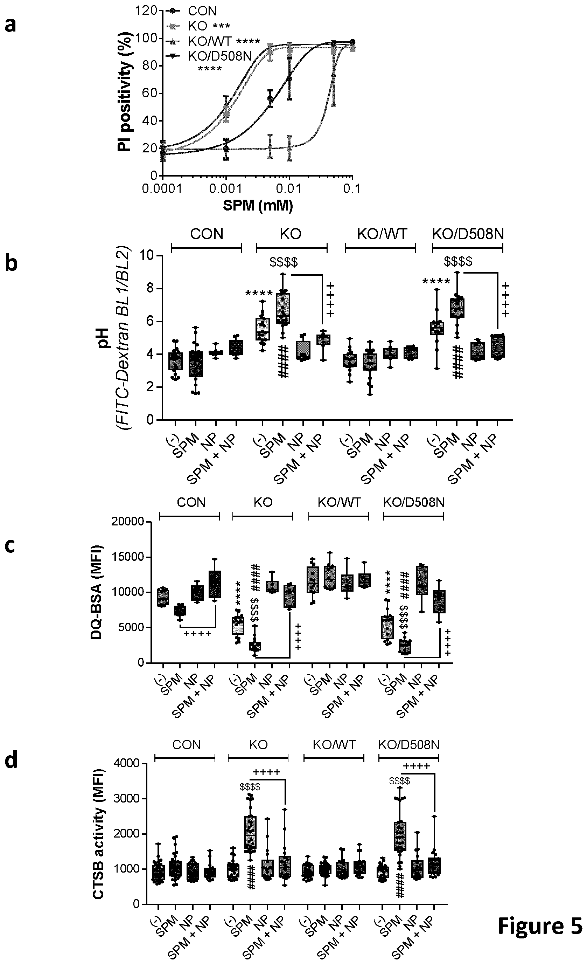

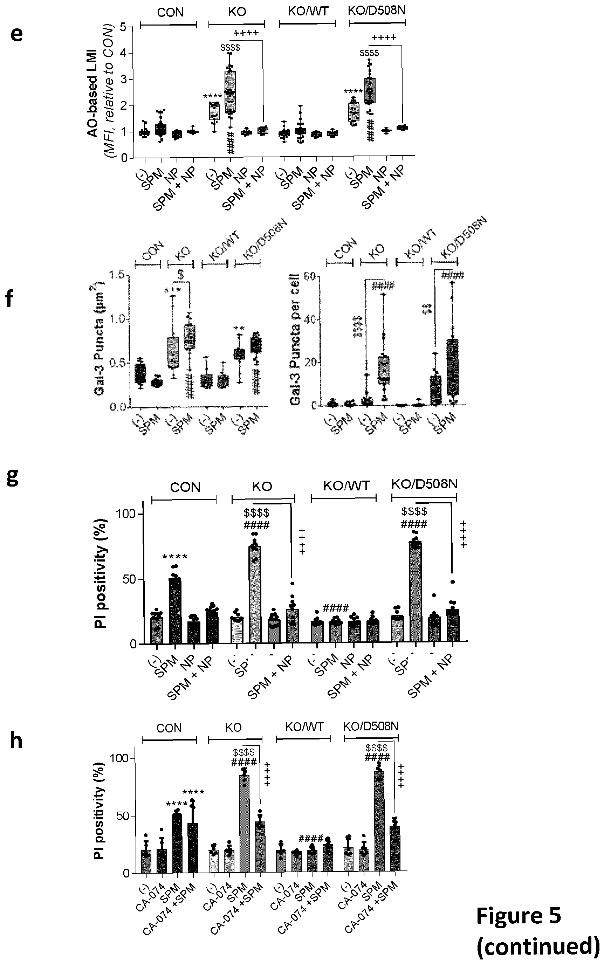

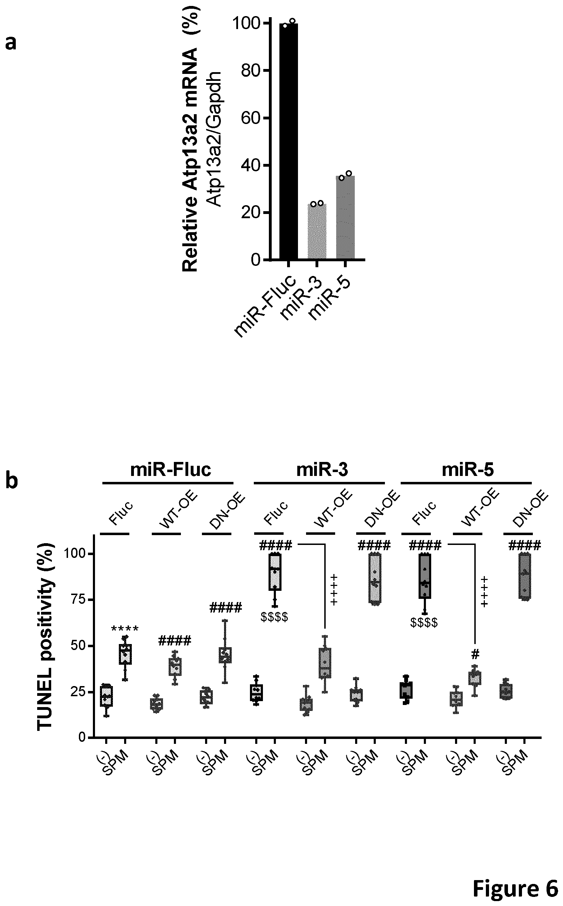

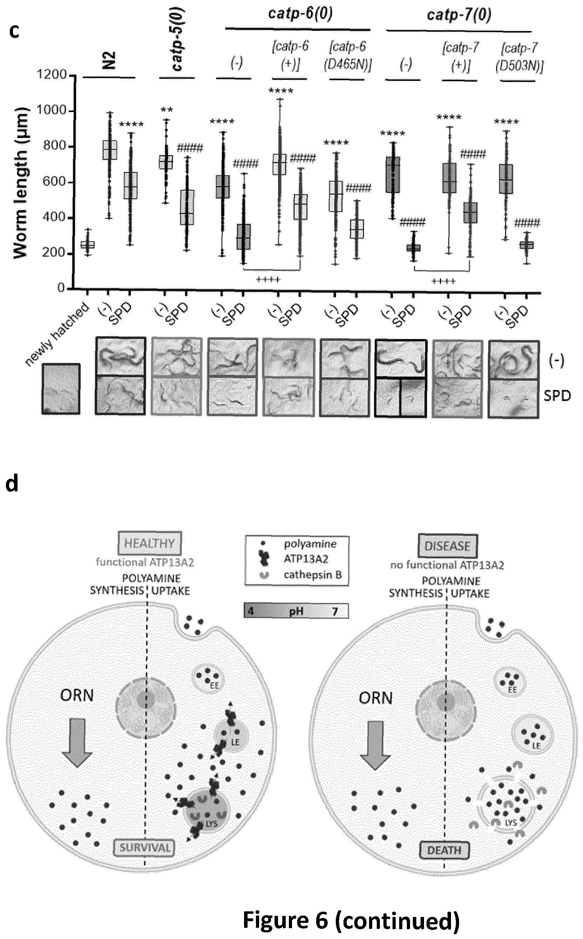

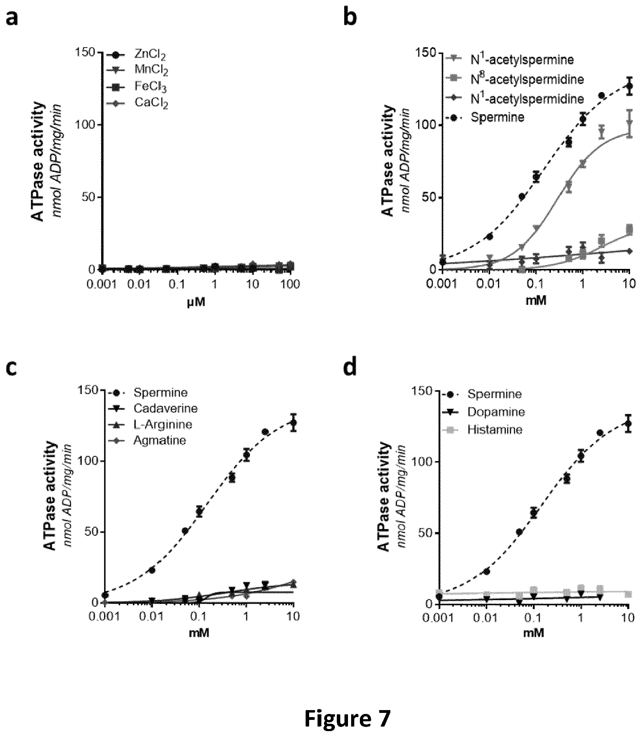

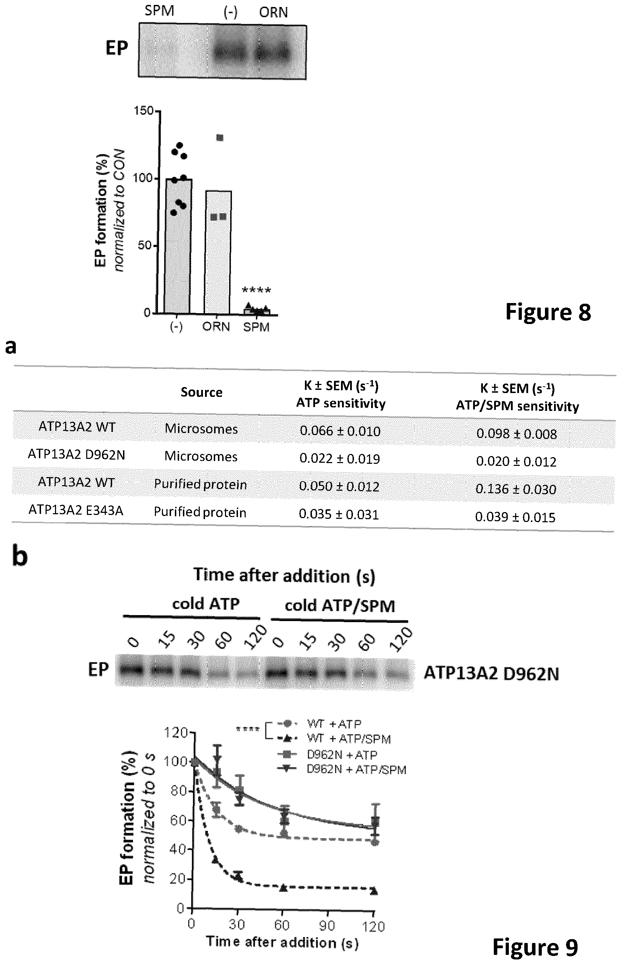

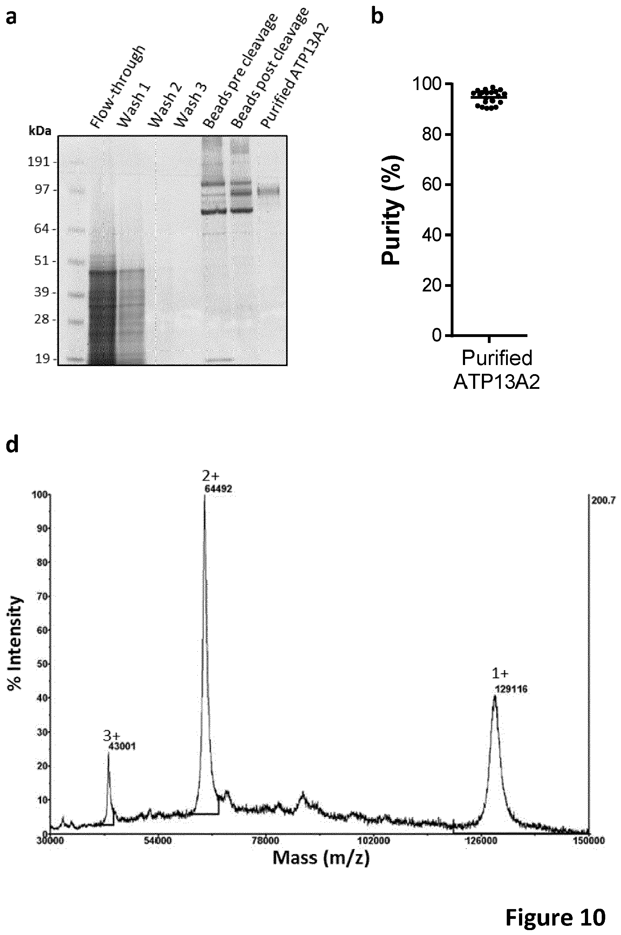

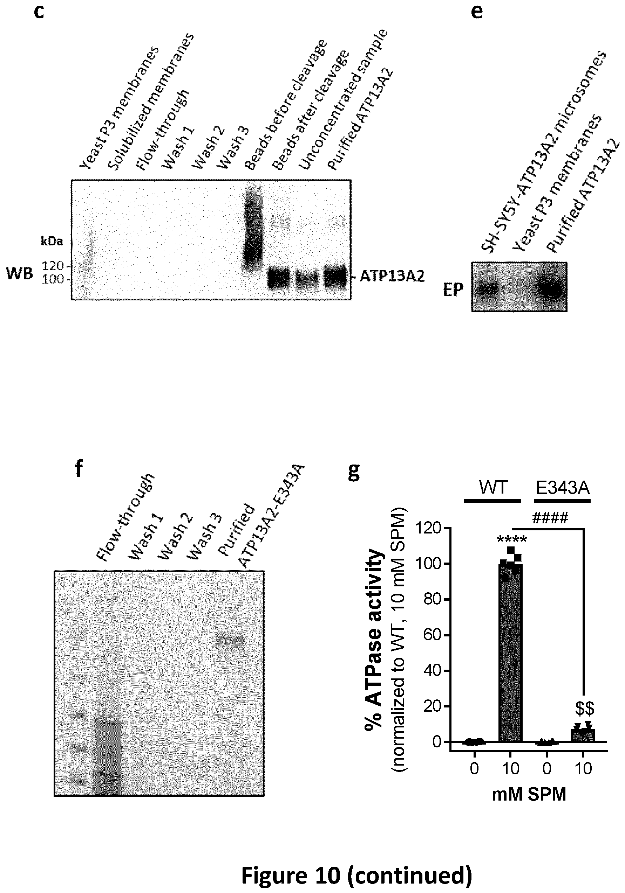

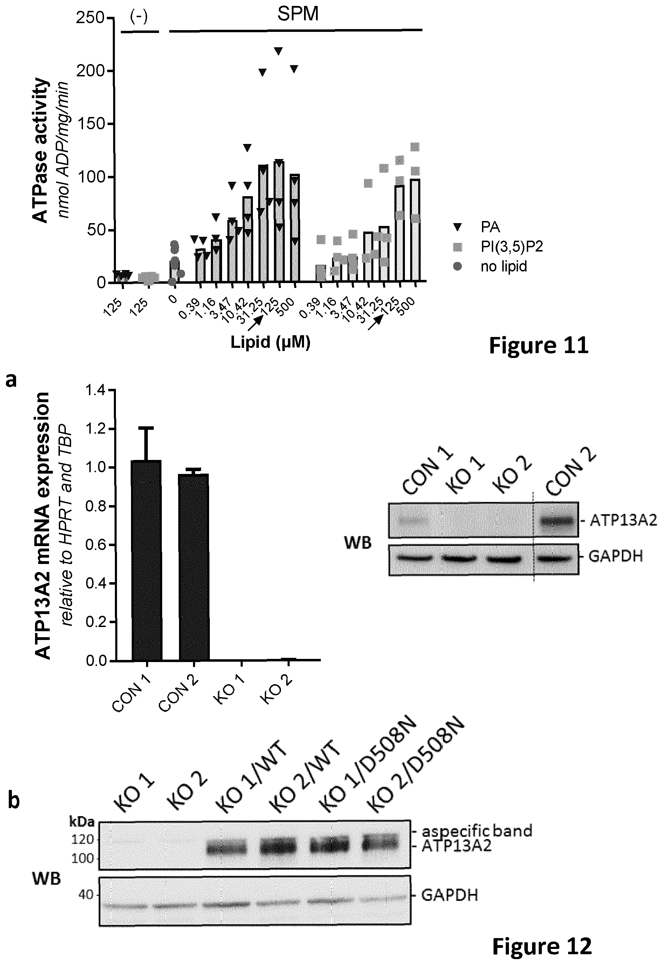

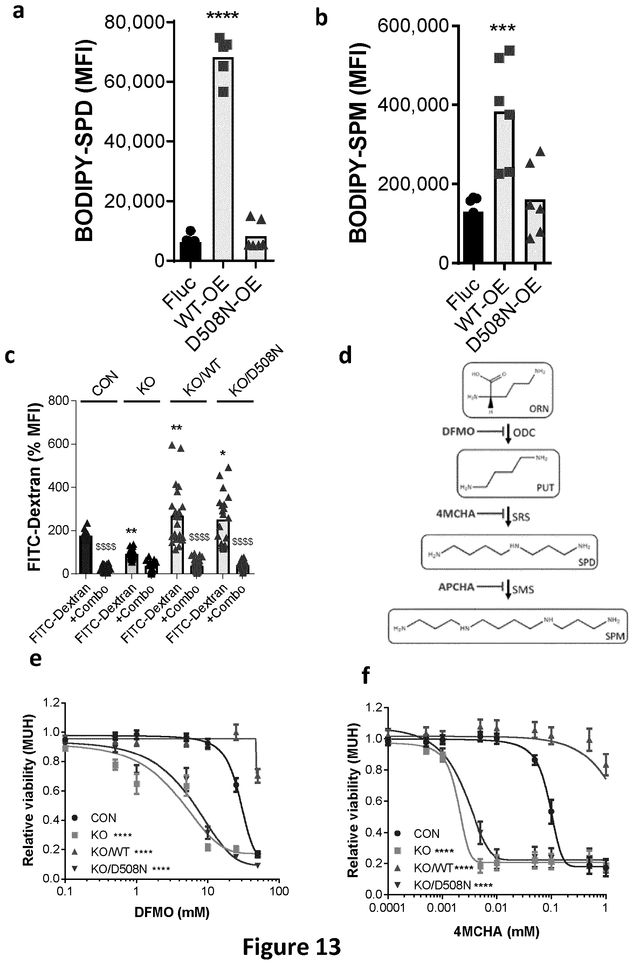

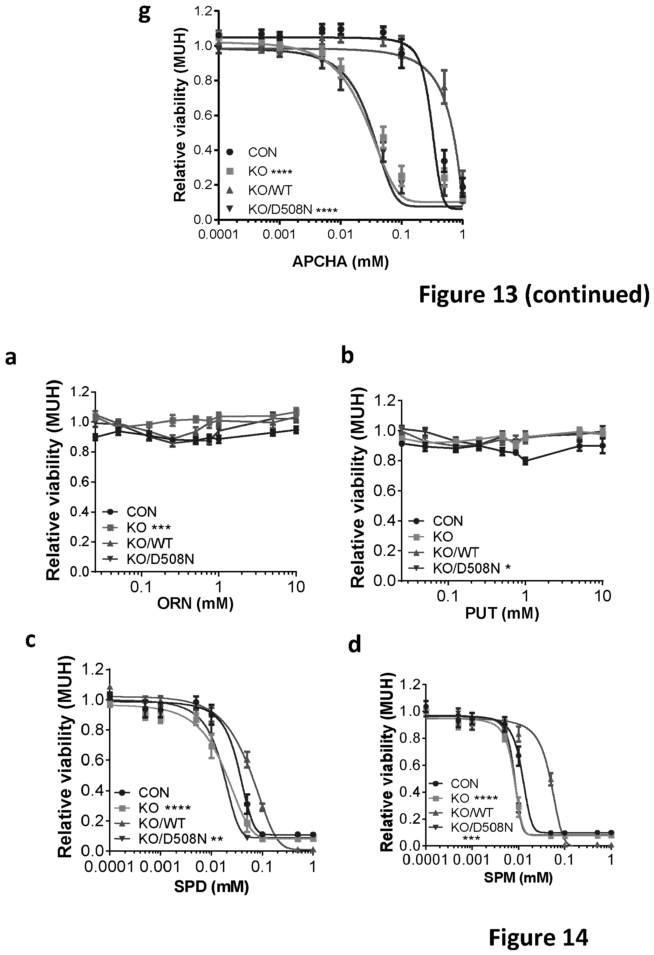

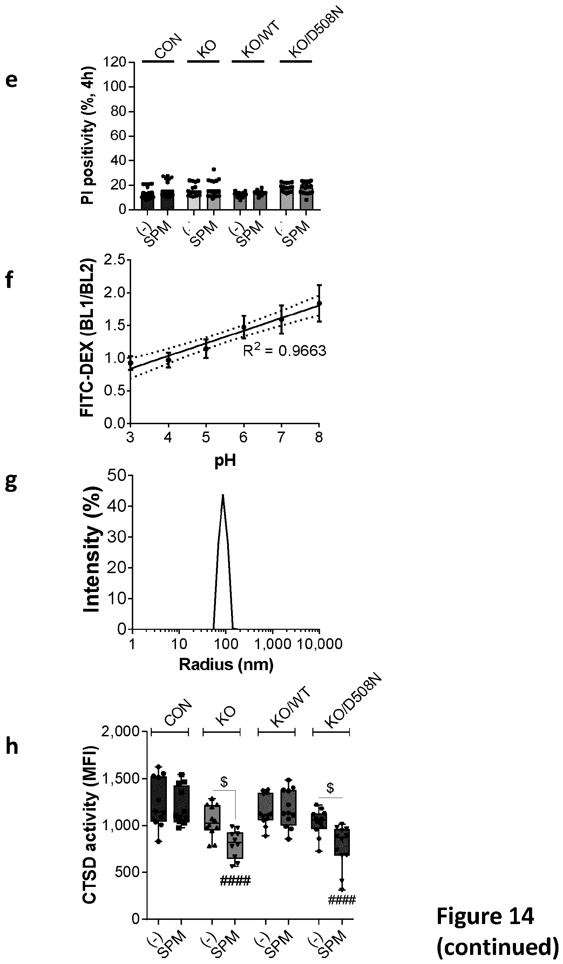

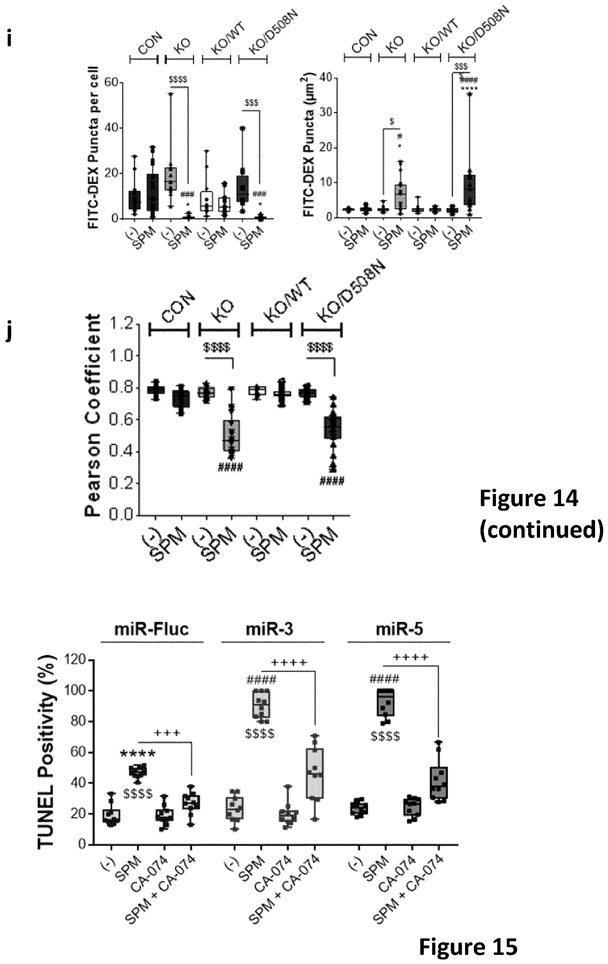

**Figure 1** - **Polyamines stimulate ATP13A2 activity. (a-b)** ATP13A2 ATPase activity was measured in solubilized microsomes (5 µg) harvested from SH-SY5Y cells that stably overexpress ATP13A2 (WT-OE or the catalytically dead D508N-OE). **(a)** A screen for candidate substrates was performed with 100 nM CaCl₂, MnCl₂, ZnCl₂ or FeCl₃ and 100 µM SPD or SPM. **(b)** Dose-response curves of ATP13A2 in the presence of the indicated doses of SPM, SPD, PUT and ORN were determined. **(c)** To evaluate the effect of SPM on phospho-enzyme formation (EP), WT-OE microsomes (20 µg) were incubated for 60 s with [γ-³²P] ATP in the presence of the indicated SPM concentrations and separated by SDS-PAGE under acidic conditions. The upper panel shows a representative autoradiogram, while the lower panel depicts the quantification of ATP13A2 phosphorylation levels. **(d)** Following 30 s of incubating WT-OE microsomes (20 µg) with [γ-³²P] ATP, the time course of dephosphorylation after an ATP chase was measured in the presence or absence of 1 mM SPM. **(e)** The ATPase activity of purified ATP13A2 WT (0.5 µg) was measured in the presence of the indicated concentrations of SPM with or without 125 µM phosphatidic acid (PA) and/or 125 µM phosphatidylinositol-(3,5)bisphosphate (PI(3,5)P2); **(f)** with the indicated doses of SPM, SPD and PUT, and 125 µM PA and PI(3,5)P2 (as a reference, we plotted the dose-response curve of SPM + PA/PI(3,5)P2 as shown in e); **(g)** or with 1 mM SPM and/or 0.25 mM orthovanadate (ORTH), a general P-type ATPase inhibitor, and 125 µM PA/PI(3,5)P2. **(h)** Purified ATP13A2 (0.5 µg) was incubated for 60 s with [γ-³²P] ATP in the presence of 1 mM SPM, and radioactivity was quantified by liquid scintillation counting. **(i)** Following 30 s of incubating purified ATP13A2 (WT or catalytically dead E343A mutant) with [γ-³²P] ATP, the time course of dephosphorylation after an ATP chase was measured in the presence or absence of 1 mM SPM. (-), vehicle treated sample. Data are represented as individual data points overlaid on group means (**a, b, j**)) or as the mean ± S.E.M (**b-g**). The values of N (independent experiments) were N = 3 (**a, b** (ORN, SPD), **d to i** (E343A)), N = 4 (**c**), N = 5 (**b** (PUT), **i** (WT)), and N = 7 (**c** (SPM)). **** *P* < 0.0001, *** P < 0.001 according to one-way ANOVA with Dunnett's test *vs.* (-) (**a, g**), two-way ANOVA with Tukey's test *vs*. WT/ATP (**d, i**) or an unpaired *t*-test *vs.* (-) (**h**). ### *P* <0.001 according to one-way ANOVA with Tukey's test *vs.* ORTH (**g**).

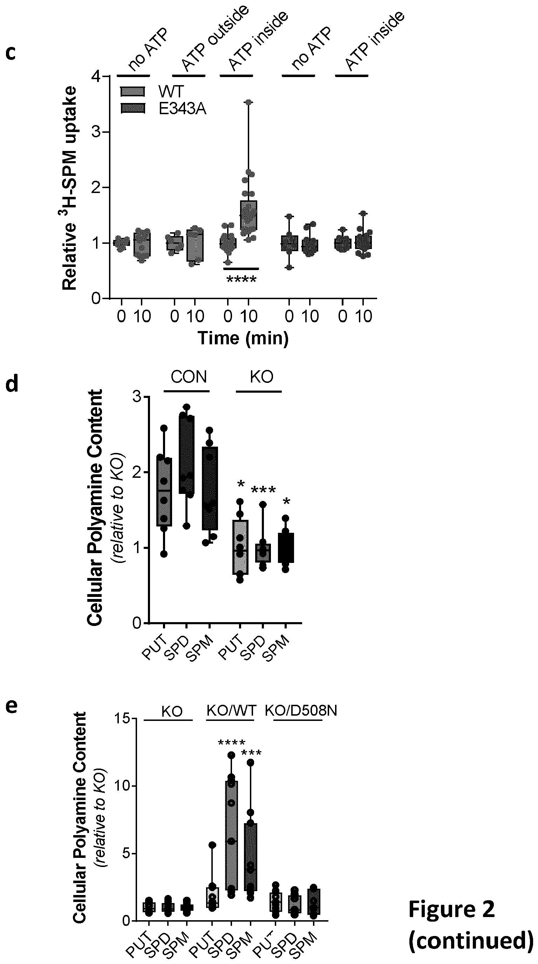

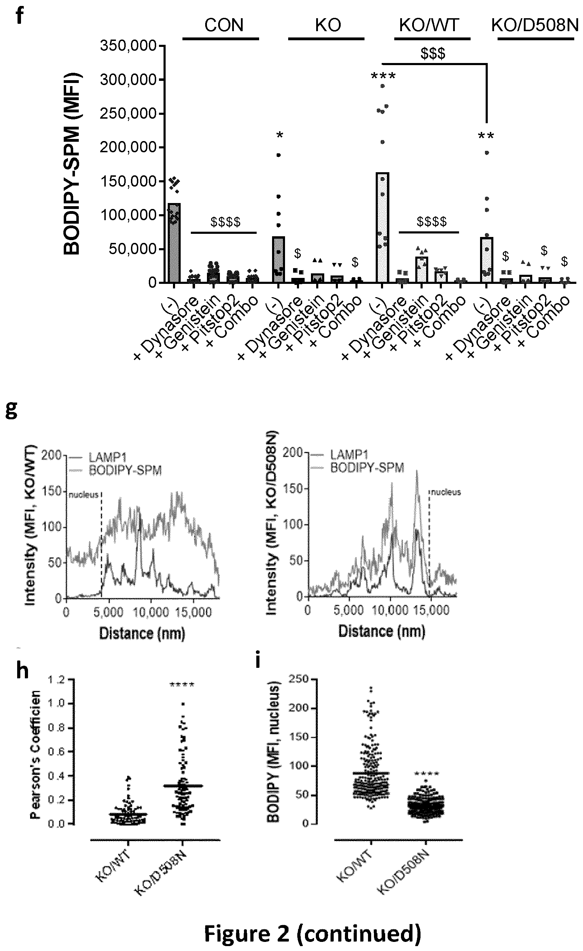

**Figure 2** - **ATP13A2 is a lysosomal polyamine exporter. (a)** Schematic illustration of vesicle reconstitution and the ³H-spermine (³H-SPM) transport assay. **(b)** Western blot analysis with primary anti-ATP13A2 antibodies (1/1,000; A3361, Sigma) and HRP-conjugated secondary antibodies (1/2,000; Cell Signaling Technology) depicts comparable ATP13A2 levels in all reconstituted vesicles. **(c)** Uptake of ³H-SPM in reconstituted vesicles derived from yeast overexpressing BAD-tagged ATP13A2 - WT or the catalytically dead mutant E343A - supplemented with phosphatidylcholine and phosphatidic acid, with or without intraluminal ATP and an ATP-regenerating system. **(d-e)** Metabolomics-based assessment of cellular putrescine (PUT), spermidine (SPD) and SPM levels in ATP13A2 KO cells (KO) *vs.* SH-SY5Y control cells (CON) with endogenous ATP13A2 **(d),** and in rescue cell lines with expression of ATP13A2 WT (KO/WT) or the catalytically dead mutant D508N (KO/D508N) on the KO background **(e). (f)** Flow cytometric assessment of the cellular uptake of BODIPY-labelled SPM. Analysis of BODIPY-SPM uptake in CON, KO, KO/WT or KO/D508N cells. The cells were pre-treated (30 min) with the endocytosis inhibitors Dynasore (100 µM), Genistein (50 µM) and/or Pitstop 2 (50 µM), alone or in combination (combo). DMSO was used as a control (-). The cells were incubated for an additional 2 h with BODIPY-SPM (**i**) at 37°C, followed by FACS analysis. All data are represented as individual data points overlaid on group means, except for the box and whisker (running from minimal to maximal values) plots in (**c, d** and **e**), for which individual data points are shown. The values of N (independent experiments) were N = 3 (**b, c** (WT ATP outside; E343A), **d, f,** N = 4 (**e**), N = 6 (c (WT no ATP) and N = 7 (**c** (WT ATP inside)). * *P* < 0.05, ** *P* < 0.01, *** *P* < 0.001, **** *P* < 0.0001 according to one-way ANOVA with Tukey's test *vs.* 0 min/ATP inside (**c**), *vs*. respective condition in CON (**d**) or KO (**e**), *vs.* CON(-) (**f**). $ *P* < 0.05, $$$ *P* < 0.001, $$$$ *P* < 0.0001 according to one-way ANOVA with Tukey's test *vs.* (-) within each cell line (**f**).
**Figure 3** - **Substrate binding site mutations reduce ATP13A2 activity. (a)** Microsomal membrane fractions were isolated from SH-SY5Y cell lines with stable overexpression of ATP13A2 WT, D508N or mutants in M4 (A467V), M6 (D962N), and M8 (K1062A). The A467V mutation converts PPALP of the predicted substrate binding site in transmembrane segment M4 into PPVLP that is present in ATP13A5^{16,69}. Also neighbouring membrane helices contribute to substrate coordination in P-type ATPases, which often relies on conserved and charged residues, such as D962 in M6 and K1062 in M8 of ATP13A2, which were also mutated¹⁶. ATP13A2 expression levels were evaluated by Western blot analysis (left: representative blot; right: bar graph depicting average expression levels) using primary anti-ATP13A2 antibodies (1/1,000; A3361, Sigma) and HRP-conjugated secondary antibodies (1/2,000; Cell Signaling Technology). **(b)** ATPase activity of ATP13A2 mutants was measured in solubilized microsomes (5 µg), and dose-response curves for spermine (SPM) were generated. As a reference, we plotted the dose-response curve of SPM, shown in Fig. 1c. **(c)** For the assessment of auto-phosphorylation, microsomes (20 µg) were incubated in the presence of 1 mM SPM. The upper panel shows a representative autoradiogram, whereas the lower panel depicts the quantification of ATP13A2 phosphorylation levels. Flow cytometric analysis of the cellular uptake of BODIPY-SPD **(d)** or BODIPY-SPM **(e)** in SH-SY5Y cells overexpressing ATP13A2 WT or the indicated mutants. The cells were incubated with 5 µM BODIPY-SPM or BODIPY-SPD for 2 h prior to flow cytometric analysis. MFI, mean fluorescence intensities; (-), vehicle treated sample. Data are represented as individual data points overlaid on group means (**a, c, d** and **e**) or as the mean ± S.E.M (**b**). The values of N (independent experiments) were N = 3 (**a**, **b, c** (D508N (SPM), A467V (SPM), D962 (SPM), and K1062A(SPM)), **d,** and **e**), N = 4 (**c** (D508N (-), A467V (-), D962N (-), and K1062A (-))), N = 5 (c (WT (-))), and N = 6 (**c** (WT SPM)). * *P <* 0.05, *** *P* < 0.001, **** *P* < 0.0001 according to one-way ANOVA with Dunnett's test *vs.* WT (**a**, **d, e**) or two-way ANOVA with Sidak's test *vs.* (-) within cell line (**c**).
**Figure 4** - **Disease-associated ATP13A2 mutants are functionally impaired. (a)** Microsomal membrane fractions were harvested from SH-SY5Y cell lines with stable overexpression of ATP13A2 mutants associated with Kufor-Rakeb syndrome (T512I and G872R) or early-onset Parkinson's disease (T12M, G528R, and A741T). ATP13A2 expression levels were evaluated by Western blot analysis (left: representative blot; right: bar graph depicting average expression levels) using primary anti-ATP13A2 antibodies (1/1,000; A3361, Sigma) and HRP-conjugated secondary antibodies (1/2,000; Cell Signaling Technology). **(b)** ATPase activity of ATP13A2 disease mutants was measured in solubilized microsomes (5 µg), and dose-response curves for spermine (SPM) were generated. As a reference, we plotted the dose-response curve of SPM, shown in Fig. 1c. **(c)** To assess auto-phosphorylation, microsomes (20 µg) were incubated in the presence of 1 mM SPM. The upper panel shows a representative autoradiogram, whereas the lower panel depicts the quantification of ATP13A2 phosphorylation levels. Flow cytometric analysis of the cellular uptake of BODIPY-SPD **(d)** or BODIPY-SPM **(e)** in SH-SY5Y cells overexpressing ATP13A2 WT or the indicated mutants. The cells were incubated with 5 µM BODIPY-SPM or BODIPY-SPD for 2 h prior to flow cytometric analysis. MFI, mean fluorescence intensities; (-), vehicle treated sample. Data are represented as individual data points overlaid on group means (**a, c**, **d,** and **e**) or as the mean ± S.E.M (**b**). The values of N (independent experiments) were N = 3 (**a** (WT, T12M, and G872R), **b** (T12M, T512I, and G872R), **c** (T12M (-), T12M (SPM), T512I (SPM), G528R (SPM), A741T (SPM), and G872R (SPM)), **d,** and **e**), N = 4 (**a** (T512I, G528R, and A741T), **b** (G528R and A741T), and **c** (WT (-), T512I (-), G528R (-), A741T (-), and G872R (-))), and N = 7 (**b** (WT), and **c** (WT (SPM))). * *P* < 0.05, *** *P* < 0.001, **** *P* < 0.0001 according to one-way ANOVA with Dunnett's test *vs.* WT (**a**, **d,** and **e**) or two-way ANOVA with Tukey's test *vs.* (-) within cell line (**c**).
**Figure 5** - **ATP13A2 protects against lysosome-dependent SPM toxicity.** The impact of exogenous polyamines on cell toxicity and lysosomal functionality was assessed in SH-SY5Y control cells (CON) with endogenous ATP13A2, ATP13A2 KO cells (KO) and rescue cell lines with WT expression (KO/WT) or expression of the catalytically dead mutant D508N (KO/D508N) on the KO background. All data represent the average of two independent CRISPR/Cas9 KO and control clones. In addition, the effect of acidic nanoparticles (NP, 100 nm diameter; Fig 15f) was evaluated. **(a)** SPM-induced cell death was analysed by propidium iodide (PI) exclusion-based flow cytometry. **(b-f)** The impact of a toxic SPM concentration (10 µM for 4 h, as determined in Fig. 5a and Fig. 14d) on lysosomal functionality was evaluated. **(b)** Lysosomal pH was measured using the fluorescent probe FITC-dextran (technical details in Methods and Fig. 14e). **(c-d)** Lysosomal degradation capacity was analysed by **(c)** DQ-BSA proteolysis (lower MFI reflects reduced lysosomal degradation as DQ-BSA normally undergoes dequenching upon proteolysis in the lysosome) as well as **(d)** cathepsin B (CTSB) activity. **(e-f)** Assessment of lysosomal membrane integrity (LMI) under basal and SPM (10 µM, 4 h) conditions, via acridine orange (AO) staining **(e),** and galectin-3 (Gal-3) punctae formation as a measure of lysosomal rupture **(f).** Box and whisker plots depict the size (bottom left) and number (bottom right) of punctae formation of confocal images of Gal-3-positive punctae with or without SPM exposure. **(g-h)** The effect of acidic NP **(g)** or the CTSB inhibitor CA-074 **(h)** on SPM-induced cytotoxicity was assessed by PI exclusion-based flow cytometry. MFI, mean fluorescence intensities; (-), vehicle treated sample. Data are represented as box and whisker (running from minimal to maximal values) plots for which individual data points are shown **(b-h)** or as the mean ± S.E.M. (**a**). The values of N (independent experiments) were N = 3 (**c** (NP, SPM + NP), **d** (NP, SPM + NP), **h**), N = 4 (**a, b** (NP, SPM + NP), **e** (NP, SPM + NP), **f, g**), N = 6 (**c** ((-), SPM), **d** ((-), SPM)) and N = 7 (**b** ((-), SPM), **e** ((-), SPM)). ** *P* < 0.01, *** *P* < 0.001, **** *P* < 0.0001 according to two-way ANOVA with Dunnett's test *vs.* CON (**a**); 1 mark *P* < 0.05, 2 marks *P* < 0.01, 3 marks *P* < 0.001, 4 marks *P* < 0.0001 according to one-way ANOVA with Tukey's (**b-e, g-h**) or Sidak's test (**f**): * *vs.* CON (-), # *vs.* CON (SPM), $ *vs.* (-) within cell line.
**Figure 6** - **Loss of ATP13A2 orthologues exacerbates polyamine toxicity in primary neurons and C. *elegans.* (a)** Atp13a2 knockdown in isolated murine cortical neurons via lentiviral miRNA transduction (miR-3 or miR-5) was confirmed by qRT-PCR. Gapdh was used as a reference gene. miR-Fluc (miRNA against Firefly luciferase), negative control. **(b)** Control (miR-Fluc) and Atp13a2 knockdown neurons were further transduced with Fluc, human ATP13A2 WT or D508N. SPM-induced cytotoxicity was assayed via TUNEL-based staining. Box and whiskers plot depicting TUNEL positivity. **(c)** *C. elegans* expresses three ATP13A2 orthologues: CATP-5-7, and we characterized single null mutants thereof *(catp-5(0), catp-5(tm4481)* X; *catp-6(0), catp-6(ok3473)* IV; *catp-7(0), catp-7(tm4438)* IV). Transgenic worms *catp-6(0);[catp-6(*+*)])* and *catp-7(0);[catp-7(*+*)]* express CATP-6 WT and CATP-7 WT in the null background, respectively; *catp-6(0);[catp-6(D465N)]* and *catp-7(0);[catp-7(D503N)]* express a catalytically dead variant in the null background.Worms were added on plates containing spermidine (SPD) and allowed to lay eggs for 8 h before being removed. Incubation of plates was continued until the N2 (WT) plates in the absence of SPD contained many mid-late stage L4s, at which point worms were rinsed off the plates. Worm lengths were determined as a read-out for toxicity. Scale bar = 100 µm. Top: quantification; bottom: representative images. **(d)** Illustration of the proposed mechanism of endo-lysosomal polyamine uptake and subsequent transfer into the cytosol via ATP13A2 (made in ©BioRender - biorender.com). (-), vehicle treated. Data are represented as the mean ± S.E.M. (**a**) or as box and whisker (running from minimal to maximal values) plots for which individual data points are shown **(b-c).** The values of N (independent experiments) were N = 2 (**a**), N = 3 (**b, c**). 1 mark *P* < 0.05, 2 marks *P* < 0.01, 4 marks *P* < 0.0001 according to one-way ANOVA with Tukey's (**b-c**): * *vs.* CON (-), # *vs.* CON (SPM), $ *vs.* (-) within condition.
**Figure 7** - **Dose-response curves of ATP13A2 ATPase activity in the presence of heavy metals, amines and polyamine analogues.** ATP13A2 ATPase activity was measured in microsomes (5 µg) harvested from SH-SY5Y cells that stably overexpress ATP13A2 WT in the presence of the indicated doses of **(a)** inorganic ions and heavy metals CaCl₂, MnCl₂, ZnCl₂ or FeCl₃; **(b)** acetylated polyamines (N¹-acetylspermine, N⁸-acetylspermidine, or N¹-acetylspermidine); **(c)** diamines (cadaverine, agmatine and amino-acid L-arginine); and **(d)** monoamines (dopamine and histamine). Data are represented as the mean ± S.E.M (N = 3). As a reference, we plotted the dose-response curve of SPM, shown in Fig. 7c.
**Figure 8** - **Ornithine does not affect ATP13A2 phospho-enzyme levels.** 20 µg of microsomes harvested from SH-SY5Y cells that overexpress ATP13A2 were incubated for 60 s with [γ-³²P] ATP in the presence of 10 mM ornithine (ORN) or spermine (SPM). Samples were subjected to SDS-PAGE (100 V, 4 h) under acidic conditions. The upper panel shows a representative autoradiogram of the phospho-enzymes (EP), while the lower panel depicts the quantification of ATP13A2 phosphorylation levels. (-), vehicle treated sample. Data are presented as individual data points overlaid on group means. The values of N (independent experiments) were N = 3 (ORN), N = 5 (SPM) and N = 8 ((-)). *****P* < 0.0001 according to one-way ANOVA with Dunnett's test *vs.* (-).
**Figure 9** - **ATP sensitivity of ATP13A2 mutants D962N and E343A is SPM-independent. (a)** Overview of rate constants of ATP13A2 phosphoenzyme decay following a chase with non-radioactive ATP with or without 1 mM SPM. **(b)** Following 30 s of incubating D962N microsomes (20 µg) with [γ-³²P] ATP, the time course of dephosphorylation after an ATP chase was measured in the presence or absence of spermine (SPM). The upper panel shows a representative autoradiogram of the phospho-enzymes (EP), while the lower panel depicts the quantification of ATP13A2 phosphorylation levels. As a reference, we plotted the WT curve, shown in Fig. 8e. Data are presented as the mean ± S.E.M (N = 4). **** *P* < 0.0001 according to two-way ANOVA with Tukey's test vs. ATP.
**Figure 10****- Streptavidin-based purification of ATP13A2 WT and catalytically dead E343A mutant. (a)** Coomassie staining showing the ATP13A2 WT purification process, starting from solubilized yeast membrane fractions, followed by streptavidin affinity chromatography and on-column thrombin cleavage to elute the protein. **(b)** Bar graph depicts protein purity as determined by densitometry of Coomassie-stained SDS-PAGE (N = 22). **(c)** Western blot analysis using a primary anti-ATP13A2 antibody (1/1,000; A3361, Sigma-Aldrich) and HRP-conjugated secondary antibodies (1/2,000; Cell Signaling Technology). **(d)** Mass spectrometry analysis of the purified ATP13A2 sample. Single, double and triple charged species are indicated. **(e)** To evaluate phospho-enzyme formation, yeast P3 membranes (20 µg) and purified ATP13A2 (1 µg) were incubated for 60 s with [γ-³²P] ATP. As a positive control, microsomes harvested from SH-SY5Y cells that overexpress ATP13A2 WT (20 µg) were used. The provided image is a representative radiogram depicting the ATP13A2 phospho-enzyme (EP). **(f)** Coomassie staining showing the ATP13A2 E343A purification process. **(g)** The ATPase activity of purified ATP13A2 WT or E343A (0.5 µg) was measured in the presence of the indicated concentrations of SPM with 125 µM phosphatidic acid and 125 µM phosphatidylinositol(3,5)bisphosphate.
**Figure 11** - **Purified ATP13A2 requires PA and PI(3,5)P2 for maximal induction of SPM-dependent ATPase activity.** The ATPase activity of purified ATP13A2 (0.3 µg) was measured in the presence of 2 mM SPM and the indicated concentrations of the ATP13A2 regulatory lipids phosphatidic acid (PA) and phosphatidylinositol(3,5)bisphosphate (PI(3,5)P2)¹. Data are represented as individual data points overlaid on group means (N = 3). Arrows indicate the concentrations of PA and PI(3,5)P2 that were used for further ATPase experiments on purified ATP13A2 (125 µM).
**Figure 12** - **Confirmation of CRISPR/Cas9-mediated ATP13A2 KO and subsequent rescue with ATP13A2 WT or D508N. (a)** The ATP13A2 KO (KO) was generated by CRISPR/Cas9 in SH-SY5Y cells and confirmed by qPCR (top) and Western blotting (bottom). ATP13A2 mRNA expression was normalized to HPRT and TBP, while GAPDH was used as a loading control for the ATP13A2 protein levels. Two fragments of the same blot were depicted and separated by a dotted line. **(b)** Rescue of ATP13A2 KO was performed by lentiviral transduction to express ATP13A2 WT (KO/WT) and the catalytically dead mutant D508N, which was used as a negative control (KO/D508N). The expression of these ATP13A2 constructs was confirmed via Western blot analysis. Data are represented as the mean ± S.E.M (N = 3).
**Figure 13** - **ATP13A2 polyamine uptake complements cytosolic polyamine synthesis. (a-c)** Flow cytometric assessment of the cellular uptake of BODIPY-labelled polyamine analogues **(a, b)** or FITC-dextran **(c).** Uptake of BODIPY-SPD **(a)** or BODIPY-SPM **(b)** in SH-SY5Y cells overexpressing firefly luciferase (Fluc, negative control), ATP13A2 WT (WT-OE) or the catalytically dead mutant D508N (D508N-OE). The cells were incubated with 5 µM BODIPY-SPM or -SPD for 2 h prior to flow cytometric analysis. **(c)** Analysis of FITC-dextran uptake (measure of endocytic capacity) was performed in SH-SY5Y control cells (CON) with endogenous ATP13A2 expression, ATP13A2 KO cells (KO) and rescue cell lines with expression of ATP13A2 WT (KO/WT) or catalytically dead mutant D508N (KO/D508N). The cells were pre-treated (30 min) with the endocytosis inhibitors Dynasore (100 µM), Genistein (50 µM) and/or Pitstop 2 (50 µM), alone or in combination (combo). DMSO was used as a control (-). The cells were incubated for an additional 2 h with FITC-dextran at 37°C, followed by FACS analysis. **(d)** Schematic representation of polyamine synthesis. ORN, ornithine; PUT, putrescine; SPD, spermidine; SPM, spermine; ODC, ornithine decarboxylase; SRS, spermidine synthase; SMS, spermine synthase. Specific inhibitors are indicated in red (ODC inhibitor DFMO, D,L-alpha-difluoromethylornithine; SRS inhibitor 4MCHA, cis-4-methylcyclohexylamine; and SMS inhibitor APCHA, N-(3-aminopropyl)cyclohexylamine). CON, KO, KO/WT or KO/D508N cells were subjected to inhibition of polyamine synthesis by DFMO **(e),** 4MCHA **(f)** and APCHA **(g)** prior to measuring cell viability via MUH (4-methylumbelliferyl heptanoate) assay. All data represent the average of two independent CRISPR/Cas9 KO and control clones. All data are represented as the mean ± S.E.M. The value of N (independent experiments) was N = 3 (**a-c**) or N = 4 (**e-g**). *P <0.05, **P< 0.01, *** P<0.001, ****P<0.0001 according to One Way ANOVA Dunnett's post hoc correction *vs*. Fluc **(a, b**) or Tukey's multiple comparison (**c**). $$$$ *P* < 0.0001 according to one-way ANOVA with Tukey's test vs. FITC-Dextran within each cell line (**c**). **** *P* < 0.0001 according to two-way ANOVA with Dunnett's test *vs.* CON (**e-g**).
**Figure 14** - **Lysosomal functionality and recovery.**
   The impact of exogenous polyamines on cell toxicity (24 h) and lysosomal functionality (4 h) was assessed in SH-SY5Y control cells (CON) with endogenous ATP13A2, ATP13A2 KO cells (KO) and rescue cell lines with WT expression (KO/WT) or expression of the catalytically dead mutant D508N (KO/D508N) on the KO background. All data represent the average of two independent CRISPR/Cas9 KO and control clones. Cytotoxicity of ornithine (ORN) **(a),** putrescine (PUT) **(b),** spermidine (SPD) **(c)** and spermine (SPM) **(d)** was assessed via a cell viability assay (4-methylumbelliferyl heptanoate (MUH)). **(e)** Cell death of the aforementioned SH-SY5Y cells was assessed after 4h SPM exposure (10 µM) by propidium iodide (PI) based flow cytometry. **(f)** Lysosomal pH was evaluated using the fluorescent probe FITC-dextran and a dual-emission ratiometric technique. FITC is excited at 488 nm and emission analyzed at 530 (BL1) and 610 nm (BL2). A pH calibration curve was generated using FITC-dextran in cells permeabilized with 100 µM monensin and equilibrated with calibration buffers (pH 3-8). **(g)** Size distribution of the acidic nanoparticles used in this study. Representative trace of three independent experiments. **(h)** Measurement of cathepsin D activity via Flexstation 3.0 microplate reader. **(i-j)** Lysosomal rupture under basal (-) and SPM (10 µM) conditions, was assessed via loss of FITC-dextran (FITC-DEX) punctae **(i),** or loss of CTSB/Lamp1 co-localization **(j).** The box and whisker plots in **(i)** depict the frequency (left) and size (right) of FITC-DEX punctae; in **(j)** the Pearson coefficient of co-localization of CTSB and Lamp1. (-), vehicle treated. Data are represented as the mean ± S.E.M. (**a-f**) or as box and whisker (running from minimal to maximal values) plots for which individual data points are shown **(h-j).** The values of N (independent experiments) were N = 3 (**e-j**) and N = 4 (**a-d**). **P* < 0.05, ** *P* < 0.01, *** *P* < 0.001, **** *P* < 0.0001 according to two-way ANOVA with Dunnett's test *vs.* CON (**a-d**); 1 mark *P* < 0.05, 3 marks *P* < 0.001, 4 marks *P* < 0.0001 according to one-way ANOVA with Dunnetts (**e**), Sidak's (**h, i** (left)) or Tukey's test (**i** (right), **j**): * *vs.* CON (-), # *vs.* CON (SPM), $ *vs.* (-) within cell line.
**Figure 15** - **Inhibition of cathepsin B activity rescues SPM-induced neuronal death.** The effect of cathepsin B inhibitor (CA-074, 10 µM) on SPM-induced (10 µM, 24 h) cell death in control (miR-Fluc) and Atp13a2 knockdown (miR-3 and miR-5) neurons was assayed via TUNEL-based staining. Left, representative confocal images; right, box and whiskers plot depicting TUNEL positivity. (-), vehicle treated. Data are represented as box and whisker (running from minimal to maximal values) plots for which individual data points are shown. The values of N (independent experiments) were N = 3. 2 marks *P* < 0.01, 4 marks *P* < 0.0001 according to one-way ANOVA with Tukey's test: * *vs.* CON (-), # *vs.* CON (SPM), $ *vs.* (-) within cell line.

One aspect of the current invention relates to in vitro methods of identifying compounds modulating (stimulating) polyamine transport into a cell. The methods comprise the steps of
a) providing a first eukaryotic cell culture wherein the endogenous P5B type ATPase gene is inactivated, wherein the cells comprise an inserted copy of a **wild type** or a **disease causing mutant** of the P5B type ATPase, and
   provding a second eukaryotic cell culture wherein the endogenous P5B type ATPase gene is inactivated, wherein the cells comprise an inserted copy of **an inactive** P5B type ATPase,
b) contacting the first and the second cell culture with a labelled polyamine and with a test compound,
c) measuring the uptake of the labelled polyamine by the first and the second cell cultures,
d) determining the difference in uptake between the two cell cultures, wherein a difference in uptake between the first and the second culture, indicates that the test compound modulates polyamine transport.
The present invention demonstrates that P5B type ATPases such as ATP13A2 transport polyamines into a cell via a two-step process, first via endocytosis and second by transport out of the endosomal system into the cytosol. Importantly, the cellular uptake capacity of polyamines correlates well with the biochemical ATPase and dephosphorylation activity of the P5B ATPase. Measuring polyamine transport can thus be used to measure P5B type ATPase activity as long as the endocytosis rate remains constant.
The methods and cells of the present invention have the advantage that there is no background signal of cells expressing wild type ATPase, this is especially useful for the cells expressing the inactive ATPase.
The present invention further allows to perform assays, without the overexpression.
Indeed cell lines are selected wherein the expression level is comparable to the expression of wild type cells, and wherein the endocytosis rate is the same.
The use of an ATPase with a disease associated mutation is particularly useful to identify test compounds which can restore ATPase activity to the level of a cell with a wild type ATPase.
The methods of the present invention allows to identify activators for wild type P5B type ATPase or disease associated mutants. These disease associated mutants are typically proteins with reduced activity. Using such a mutant allows to screen for compounds which restore the activity of the mutant protein. An inactive protein is used as control.
When a wild type protein is used to screen for activators of this wild type protein, the control can be a complete or partially inactivated P5B type ATPase.
Typically the P5B type ATPase is ATP13A2.
"P5B type ATPase" of the present invention refers to ATP13A2, ATP13A3, ATP13A4 or ATP13A5.
In these methods, the inactive ATPase has no autophosphorylation, dephosphorylation, ATPase and/or transport activity.
Herein Inactive ATPase refers to mutated and /or truncated version of the protein wherein polyamine transport is absent, autoposphorylation is lacking, dephosphosphoryation of other proteins is absent, and/or lacks the capacity to convert ATP into ADP.
Examples of inactive ATP13A2 , are the D508N or D962N mutant, with reference to the numbering of ATP13A2 variant 2. Numerous other mutants have been described that are associated with diseases. Genetic variants are disclosed e.g. in the OMIM database.
ATP13A2 mutants lacking cellular polyamine uptake and biochemical ATP activity include D508N, D962N, T512I and G672R. The mutant E343A is defective in defosforylation and has no ATPase activity.
A multiple sequence alignment allows to identify the corresponding mutations in other P5B type ATPase
Typically the eukaryotic cells used in these methods are mammalian cells. The invention is illustrated using human cells. However, the methods can be equally performed using other mammalian cells, such as mice, rat, using cells of non-mammalian vertebrate such as e.g. zebrafish, cells of invertebrates such as c. *elegans* or eukaryotic cells such as yeast.
In alternative embodiments, the ATPase is inactivated in cells of first organism and transfected with an ATPase of a second organisms.
The methods of the invention can be used for identifying compounds stimulating polyamine transport.
In the methods of the present invention, the labelled polyamine is a typically a fluorescently labelled polyamine.
The methods of the present invention can be performed with any detectable label (e.g. chromophoric or radioactive) to the extent that the label does not disturb the uptake of the polyamine or is not lethal to the cell. The examples of the present invention demonstrate that the presence of a fluorescence group which is coupled to the polyamine does not impede the uptake of the polyamine. The suitability of other detectable labels can be determined by comparison with the fluorescent labels as used in the examples.
Generally the uptake of a fluorescent polyamine is determined by FACS analysis of cells.
Other methods to detect the uptake of a label by a cell are for example readers of multiwell plates. Alternatively, fluorescence microscopy can be used to detect polyamine accumulation by cells as a whole, by reduction in lysosomal fluorescence using co-localization with lysosomal dye, or by an increase in cytosolic/nuclear or mitochondrial fluorescence using co-localization with organelle specific dyes
In specific embodiments prior to the analysis, extracellular fluorescent polyamine is washed away or quenched.
Generally, the polyamine used in the methods of the present invention is putrescine, spermine or spermidine.
Other polyamines, or modified version can be equally tested, by comparison with one of putrescine, spermine or spermidine, or by applying the polyamines in the above described assay in the absence of a test compound.
In specific embodiments of the present invention cells are further subjected to oxidative stress. Examples of compounds inducing oxidative stress are mitochondrial complex I inhibitors such as Rotenone, MPP+, MPTP, 6-OHDA, rhodamine-6G or heavy metals such as MnCl₂, FeCl₃ and ZnCl2.
In alternative embodiments, cells are subjected to polyamine synthesis pathway, such as the ornithine decarboxylase inhibitor Difluoromethylornithine (DFMO), thereby limiting polyamine access to the exogenous administered labelled polyamine.
As an alternative or in addition to the cellular uptake of the labelled polyamine, cell survival or cell death is determined. At high concentrations (above 10 µM) polyamines are toxic to cells, and the degree of cell toxicity is related to the polyamine uptake activity of the P5B ATPase. Cell death or cell viability can be determined e.g. by uptake or removal of dyes. Cell death can be measured by propidium iodide, caspase-3 cleavage or a TUNEL assay. Cell survival can be determined by an MUH assay.

In addition or alternatively to measurement of labelled polyamines, polyamine transport can also be measured by determining lysosomal functionality.
The P5B ATPase activity determines the endo-/lysosomal polyamine content which affects lysosomal functionality, where a high P5B ATPase activity improves lysosomal functionality. Lysosomal functionality can be determined for example by measuring intracellular pH (using a pH sensor such as LysoTracker or FITC-dextran), lysosomal degradative capacity (cathepsin activity or DQ-BSA turnover) or lysosomal membrane integrity (cytosolic FITC-dextran or cathepsin B release, reduction in lysosomal acridine orange uptake, or Gal-3 punctae formation).
The methods of the present invention can be used for detection of components activating P5B type ATPase having neuroprotective properties.
The methods of the present invention can be used, for detection of components inhibiting P5B type ATPase having antitumor properties.
The methods of the present invention can be used for detection of components activating P5B Type ATPase, having cardiovascular activity. Indeed, a role of ATP13A3 in pulmonary arterial hypertension has been is described.

The methods of the present invention are performed with specific cell lines. These cell lines are a further aspect of the invention.
Accordingly the present invention relates to an eukaryotic, typically mammalian, cell line wherein the endogenous P5B type ATPase gene is inactivated, **characterised in that** the cell line comprises an inserted copy of a wild type or mutant P5B type ATPase.
Alternatively, endogenous P5B type ATPase is mutated to an inactive variant by genome editing (e.g. CRISPR/Cas) instead of re-insertion of a copy in a knock out background.
Typically the endogenous P5B type ATPase gene is inactivated or mutated by crispr/cas.
The inserted copy can delivered via a viral vector, such as an adenoviral vector, an AAV vector, or a lentiviral vector.
The methods of the present invention are performed with the above cell lines wherein one cell line has an inactive P5B type ATPase. The combination of cell lines are a further aspect of the invention.
The invention thus relates to a combination of cells and their use in screening methods as described in the present invention. The cell lines are referred to as a kit

Of a first eukaryotic (mammalian) cell line wherein the endogenous P5B type ATPase gene is inactivated, characterised in that the cell line comprises an inserted copy of a **wild type or disease causing mutant** P5B type ATPase, and
a second eukaryotic (mammalian) cell line wherein the endogenous P5B type ATPase gene is inactivated, characterised in that the cell line comprises an inserted copy of a an inactivated P5B type ATPase.

In this kit the first and the second cell line have comparable expression levels of said inserted ATPase.
Herein comparable expression levels refers to a minor difference in expression level of at most 25 %, at most 20 %, at most 10% or at most 5% as determined by a quantitative immunoassay or mass-spectrometry-based analysis.
In this kit the first and the second cell line have comparable endocytosis activity. Herein comparable endocytosis activity refers to a difference in endocytosis activity of at most 25 %, at most 20 %, at most 10% or at most 5% as determined by FITC-dextran uptake capacity or a comparable method.
In this kit the inactivated P5B type ATPase is a catalytic inactive mutant lacking autophosphorylation, dephosphorylation, ATPase or transport activity.
An example of a catalytic inactive mutant is the D508N mutant, with reference to the numbering of ATP13A2 variant 2. Other suitable mutants include T512I and G872R
The cells lines in the kit can be primary or immortalised brain cell lines, for example a neuroblastoma cell line.
In addition to the above described assays wherein cell lines are used, the invention relates to methods wherein no cell are used.
In another aspect, the invention relates to an in vitro method of screening compounds modulating P5B type ATPase activity comprising the steps of:
a) providing a cell membrane fraction of cells expressing wild type P5B type ATPase or a disease causing mutant thereof in a buffered solution comprising:
   - polyamine as transported substrate
   - ATP
   - a solubilising or membrane permeabilizing agent
   - and a test compound
b) measuring ATP hydrolysis (by e.g. luminescence by measuring the released ADP, or colorimetric by measuring inorganic phosphate production)
c) comparing the measured ATP hydrolysis with the value of a control experiment without test compound, with vehicle control or with reference compounds that are modulators of P5B ATPase or polyamine transport activity.
d) wherein a difference in ATP hydrolysis is indicative of a test compound modulation P5B type ATPase activity .
In this method a solubilising or membrane permeabilizing agent provides access of the polyamine to the luminal compartment (e.g. non-denaturing or non-ionic detergents like n-Dodecyl β-D-maltoside (DDM), C12E8, LMNG, Triton-X100, ...; nanodiscs; or styrene maleic acid copolymers).
This method differs essentially from the prior art of De La Hera, wherein membranes were not solubilized and access of the polyamines to the luminal compartment is therefore prevented, hampering the measurement of polyamine-dependent P5B ATPase activity.
As such the methods of De La Hera could not be reproduced. The problem to assay P5B type ATPase activity have been solved by disrupting the membrane integrity of the vesicles.
In this method different controls can be envisaged:
Regulatory lipids such as phosphatidic acid and/or phosphatidylinositol (3,5) bisphosphate interact with P5B-ATPase and prime the enzyme for activation.
Using such lipid activated enzyme as control allows to screen for activating compounds.
P-type ATPase or polyamine transport inhibitors can be used as controls to screen for inhibitors. A first type of control inhibitor could be a general P-type ATPase inhibitor, such as orthovanadate, which is a general inhibitor of all P-type ATPases that is confirmed to block P5B ATPases. A second type of control inhibitor could be a general polyamine transport inhibitor, such as benzylviologin.
Identified compounds should have no effect on the primary assay in the absence of P5B ATPase (counterscreen).
Yet another aspect of the present invention is a
A method of screening compounds modulating recombinant and purified P5B ATPase activity comprising the steps of:
a) Providing a buffered solution comprising solubilised purified P5B-ATPase such as ATP13A2
   - a polyamine
   - ATP
   - and a regulatory lipid or agent that targets the auto-regulatory domain of the P5B-ATPase
b) adding a test compound,
c) determining ATPase activity, and comparing the determined active with the activity of a control experiment without test compound, with vehicle control or with reference compounds that are modulators of P5B ATPase or polyamine transport activity.
As an alternative to the assays using cell lines or using a membrane fraction comprising P5B ATPase a recombinant and purified ATPase can be used. Compounds such as a detergent, nanodisc or styrene maleic acid copolymer can solubilize purified P5B-ATPase to provide access of the polyamine to the luminal side of the enzyme while preserving coupled ATPase activity.
Suitable examples of detergents are non-denaturing or non-ionic detergents, such as n-Dodecyl β-D-maltoside (DDM), C12E8, LMNG and Triton-X100.
Regulatory lipids such as phosphatidic acid and/or phosphatidylinositol (3,5) bisphosphate interact with P5B-ATPases and prime the enzyme for activation. Using such lipid activated enzyme as control allows to screen for activating compounds.
P-type ATPase or polyamine transport inhibitors can be used as controls to screen for inhibitors. A first type of control inhibitor could be a general P-type ATPase inhibitor, such as orthovanadate, which is a general inhibitor of all P-type ATPases that is confirmed to block P5B ATPases. A second type of control inhibitor could be a general polyamine transport inhibitor, such as benzylviologin.
Identified compounds should have no effect on the primary assay in the absence of P5B ATPase (counterscreen).

The present invention demonstrates that polyamines stimulate ATP13A2 activity P-type transporters couple ATP hydrolysis to the binding of the proper substrate for transport¹⁶. To screen for the transported substrate(s) of ATP13A2, we turned to solubilized microsomal membrane fractions of SH-SY5Y cells that overexpress active and high levels of human ATP13A2 wild type (WT) (WT-OE) or comparable levels of the catalytically dead D508N mutant (D508N-OE)^{7,12}, and measured ATPase activity in the presence of various candidate substrates.
ATP13A2 is generally referred to as a putative manganese²⁶, zinc^{20,27} or iron²⁸ transporter, but MnCl₂, ZnCl₂ or FeCl₃ did not increase ATP13A2 activity (Fig. 1a, Fig. 7a). Additionally, CaCl₂ had no effect (Fig. 1a, Fig. 7a). In contrast, the polyamines SPD (spermidine) and SPM (spermine) significantly stimulated ATPase activity of ATP13A2 WT, whereas SPM had no effect on the D508N mutant (Fig. 1a), indicating that polyamines may represent the transported substrate class. Diamines (putrescine (PUT), cadaverine, agmatine and acetylated SPD), monoamines (dopamine and histamine) and amino acids (L-arginine and ornithine, ORN) did not induce ATP13A2 activity (Fig. 1b; Fig. 7b-d), while the polyamines SPM, N¹-acetylspermine, and SPD were able to stimulate ATPase activity in a concentration-dependent manner (Fig. 1b, Table 1, Fig. 7b). ATP13A2 presents the highest apparent affinity for SPM (Kₘ = 149 ± 34 µM), which falls within the physiological concentration range of cellular polyamine levels (10-1000 µM)²⁹. ATP13A2 displays a maximal ATPase turnover rate (Vₘₐₓ) of 140 ± 6 nmol ADP/mg/min, while the Hill coefficient of SPM-induced ATPase activity approaches one, suggesting a 1:1 SPM:ATP stoichiometry.

**Table 1 Overview of the apparent Km and Vmax values for ATP13A2 in the presence of relevant polyamines**

| | Km ± SEM (µM) | | Vmax ± SEM (nmol ADP/mg/min) | |
|---|---|---|---|---|
| | Microsomal ATP13A2 | Purified ATP13A2 | Microsomal ATP13A2 | Purified ATP13A2 |
| Spermine | 149 ± 34 | 76 ± 26 | 140 ± 6 | 159 ± 10 |
| N1-acetylspermine | 286 ± 48 | not tested | 98 ± 5 | not tested |
| Spermidine | ∼ 1700* | 890 ± 501 | ∼ 106* | 194 ± 26 |

| | | | | |
|---|---|---|---|---|
| * estimated values (could not be accurately determined following fitting of the curve) | | | | |

The catalytic auto-phosphorylation and/or dephosphorylation reactions of P-type ATPases occur in response to binding of the proper transported substrate¹⁶. ATP13A2 forms a phospho-intermediate on the D508 residue in the absence of SPM supplementation^{12,13} and auto-phosphorylation is not stimulated further by SPM (Fig. 1c). Instead, SPM leads to a dose-dependent reduction in ATP13A2 phospho-enzyme levels (Fig. 1c), which is not seen with ORN (Fig. 8). This suggests that SPM may stimulate the dephosphorylation reaction, which is confirmed by the significantly higher dephosphorylation rate in the presence of SPM following a chase with non-radioactive ATP (Fig. 1d, Fig. 9a). Substrate-induced dephosphorylation has also been reported for other P-type ATPases like Na⁺/K⁺-ATPase (P2 subfamily) and lipid flippases (P4 subfamily)¹⁷, further indicating that SPM may be the *bona fide* transported substrate.
SPM directly modulates the activity of purified human ATP13A2, which was obtained from a yeast expression system (as described for other P-type ATPases³⁰). Following biotin acceptor domain (BAD)-based affinity chromatography and subsequent cleavage of the tag, we obtained >95% pure, full length and solubilized ATP13A2 (Fig. 10a-d) that retained the ability to form a phospho-intermediate (Fig. 10e). Importantly, SPM stimulated the ATPase activity of purified ATP13A2 (Fig. 1e), although the apparent SPM affinity was considerably lower (Kₘ = 1.06 ± 0.94 mM) than that of microsomes (Table 1). However, ATP13A2 activity was strongly stimulated by the regulatory lipids phosphatidylinositol (3,5) bisphosphate (PI(3,5)P2) and phosphatidic acid (PA), which relieve auto-inhibition by binding to the ATP13A2 N-terminus¹¹⁻¹³. These lipids stimulate ATPase activity only in the presence of SPM, with a maximal effect at 125 µM (Fig. 11). When both lipids were supplied together, purified ATP13A2 acquired properties and substrate specificities similar to those observed in microsomes (SPM: Kₘ = 76 ± 26 µM, Vₘₐₓ = 159 ± 10 nmol ADP/mg/min; SPD: Km = 890 ± 501 µM, Vₘₐₓ = 194 ± 26 nmol ADP/mg/min) (Fig. 1e-f, Table 1). In addition, SPM-induced ATPase activity was blocked by orthovanadate (ORTH), a general P-type ATPase inhibitor¹⁷ (Fig. 1fg). Finally, we also purified the ATP13A2 mutant E343A, carrying a mutation in the conserved catalytic motif for dephosphorylation (³⁴¹TGES). E343A was expressed and purified as WT, undergoes auto-phosphorylation (Fig. 1g), but displays no SPM-induced ATPase activity (Fig. 10f-g). Importantly, when the phosphoenzyme is chased with cold ATP, SPM clearly stimulates dephosphorylation of purified WT, but not of the E343A mutant (Fig. 1h-i, Fig. 9a).
Taken together, our results demonstrate that SPM induces ATPase activity and the catalytic dephosphorylation reaction of purified ATP13A2, suggesting that ATP13A2 is a SPM transporter.

The present invention demonstrates that ATP13A2 is a lysosomal SPM exporter that promotes cellular SPM uptake.
Substrate-induced dephosphorylation is characteristic of P-type ATPases that transport their substrates from the exoplasmic to the cytosolic side of membranes, such as K⁺ in Na⁺/K⁺-ATPase or lipids in lipid flippases³¹. To verify whether ATP13A2 transports SPM in the same direction, we carried out transport assays with ³H-labelled SPM (³H-SPM) in reconstituted vesicles from solubilized yeast membranes expressing BAD-labelled ATP13A2 WT or E343A, supplemented with the activating lipid PA. This reconstitution renders two populations of ATP13A2 proteins that are either inserted right side out (ATP-binding domain in the extra-luminal space) or inside-out (ATP-binding domain in the lumen) (Fig. 2a).
Immunoblotting confirmed comparable levels of ATP13A2 WT or E343A in reconstituted vesicles (Fig. 2b). To activate ATP13A2, ATP was first supplied outside the vesicles, but no ³H-SPM uptake was observed for ATP13A2 WT ((1) in Fig. 2a; Fig. 2c). Importantly, uptake of ³H-SPM into vesicles was detected for ATP13A2 WT, but not the E343A mutant, when ATP was present inside the vesicles, together with an ATP-regenerating system (Fig. 2c), *i.e.* when ATP13A2 was positioned inside-out ((2) in Fig. 2a). Extending these insights to the cellular context, where ATP13A2 is present in the late endo-lysosomal compartment^{4,32}, ATP13A2 most likely operates as a lysosomal SPM exporter.
Next, we assessed whether endogenous ATP13A2 influences the cellular polyamine content. Via CRISPR/Cas9 genome editing in SH-SY5Y cells, we generated two independent ATP13A2 knock-out cell lines (KO, Fig. 12a). Importantly, we demonstrated via mass spectrometry³³ that the total cell content of PUT, SPD and SPM was significantly lower in KO than in control (CON) cells (Fig. 2d). The KO cells were subsequently rescued by overexpression of ATP13A2 WT (KO/WT), whereas the D508N mutant was expressed as a transport-deficient control (KO/D508N) (Fig. 12b). Consequently, the total cell content of SPM and SPD was significantly higher in KO/WT cells than in KO or KO/D508N cells (Fig. 2e). Thus, ATP13A2 enhances the cellular polyamine pool by uptake of polyamines from the extracellular environment in an activity-dependent manner.
In view of the late endo-lysosomal localization of ATP13A2^{4,32}, we explored whether ATP13A2 increases the polyamine content in cells via endocytosis, a polyamine uptake route that was suggested previously³⁴. Via flow cytometry, we measured levels of BODIPY-labelled analogues that are taken up in the cell via the polyamine transport system³⁵. WT-OE cells took up significantly more BODIPY-SPD (Fig. 13a) and BODIPY-SPM (Fig. 13b) than D508N-OE or control cells (expressing firefly luciferase, Fluc). Similarly, KO/WT cells presented a twofold higher BODIPY-SPM uptake than ATP13A2 KO and KO/D508N cells, which took up less BODIPY-SPM than CON cells (Fig. 2f). The comparable FITC-dextran uptake in the KO/WT and KO/D508N cell lines (Fig. 13c) further showed that the increased BODIPY-SPM uptake in KO/WT *versus* KO/D508N is not merely the result of an increased endocytic rate, but depends on the transport activity of ATP13A2. The stimulatory effect of ATP13A2 on endocytosis appears as a transport-independent phenotype, as reported previously^{36,37}. Thus, we can rely on BODIPY-SPM uptake as a readout to assess ATP13A2 transport activity in cells. Since ATP13A2 transports SPM towards the cytosol (Fig. 2c) and increases the cellular SPM content (Fig. 2d-e), ATP13A2 may stimulate SPM uptake via endocytosis before ATP13A2 actively exports the internalized pool to the cytosol. Indeed, endocytosis inhibitors not only prevented FITC-dextran uptake (Fig. 13c) but also blocked BODIPY-SPM uptake in KO/WT cells (Fig. 2f). Additionally, CON cells and to a lesser extent KO and KO/D508N cells also displayed capacity to take up BODIPY-SPM, which was prevented by endocytosis blockers (Fig. 2f). Via confocal microscopy, we confirmed the higher cellular BODIPY-SPM content in KO/WT cells as compared to their transport-deficient counterparts KO/D508N (Fig. 2g). Moreover, we also assessed the intracellular distribution of BODIPY-SPM in both cell models (Fig 2h-k). In the KO/D508N cells BODIPY-SPM mainly co-localized with LAMP1 positive vesicles (Fig. 2h-j), indicating that in the absence of active ATP13A2 polyamines mainly accumulate in the late endo-lysosomes. In contrast, cells with functional ATP13A2 displayed a broader distribution of BODIPY-SPM (Fig. 2h-k), which was more abundant outside vesicular compartments, such as in the cytosol and nucleus (Fig. 2i, k). These observations confirm the biochemical activity and transport direction of ATP13A2 in a cellular context.
Thus, polyamines enter the cell via endocytosis, irrespective of ATP13A2, but when functional ATP13A2 is present in the late endo-lysosomes, more polyamines can be taken up and redistributed as a consequence of the lysosomal polyamine export function of ATP13A2.
By stimulating cellular SPM uptake and transporting SPM into the cytosol, ATP13A2 may complement endogenous SPM synthesis, which critically depends on the rate-limiting enzyme ornithine decarboxylase (ODC) (Fig. 13d). Indeed, KO/WT cells were protected against pharmacological inhibition of ODC, while a lack of ATP13A2 activity (KO or KO/D508N) sensitized cells to ODC inhibition (Fig. 13e), in line with the negative genetic interactions between ATP13A2 orthologues and ODC in yeast³⁸ and *Caenorhabditis elegans*²⁴. ATP13A2 also protected against toxicity mediated by other polyamine synthesis inhibitors that target either SPD (Fig. 13f) or SPM synthase (Fig. 13g). Thus, ATP13A2 complements a reduction in SPM and SPD synthesis, which is consistent with the cytosolic import of SPM and/or SPD by ATP13A2.
Taken together, ATP13A2 promotes cellular uptake of SPM and SPD by transporting these polyamines from the late endo-lysosomal compartments to the cytosol.
The present invention demonstrates that substrate binding site and disease mutations reduce SPM-induced ATP13A2 activity
All P-type ATPases contain a binding site for the transported substrate that positions around transmembrane segment M4. Via mutagenesis, we examined whether the stimulatory effect of SPM on ATP13A2 activity depends on residues in the predicted substrate binding site. We generated stable SH-SY5Y cell lines that overexpressed comparable levels of ATP13A2 WT, D508N or three mutants in the membrane region (A467V on M4, D962N on M6 and K1062A on M8) (Fig. 3a; rationale for residue choice in legend). Importantly, the three mutants displayed a lower SPM-induced ATPase activity and apparent SPM affinity (Fig. 3b), but also a reduced cellular uptake of BODIPY-SPD/SPM (Fig. 3d-e). Consistent with the ATPase activity, the SPM-induced dephosphorylation is completely abolished in the D962N mutant (Fig. 3c and Fig. 9a-b). D962 in the membrane may possibly serve as a SPM sensor that couples SPM binding to the dephosphorylation reaction in the cytosolic domains. Together, we identified residues in or near the predicted substrate binding site of ATP13A2 that are important for SPM-dependent ATPase, dephosphorylation and transport activities that may contribute to SPM coordination in the membrane region.
To date, over thirty disease-associated mutations in *ATP13A2* have been reported in a spectrum of neurodegenerative disorders^{7,8,25}. Here, we evaluated the functional impact of non-truncating and mostly lysosomal located variants linked to two ATP13A2-associated diseases, EOPD (T12M⁵, G528R⁵ and A741T³⁹⁻⁴¹) or KRS (T512I^{14,15,42} and G872R⁴³). We generated stable SH-SY5Y cell lines with comparable expression of WT ATP13A2 or disease mutants (Fig. 4a). The KRS mutants T512I and G872R did not exhibit ATPase (Fig. 4b) or auto-phosphorylation activity (Fig. 4c) and were insensitive to SPM. The effect of the EOPD mutations T12M, G528R and A741T was less severe, although a reduction in the apparent SPM affinity was observed (Fig. 4b). Phospho-enzyme levels of the EOPD mutants were more (T12M) or less (G528R, A741T) sensitive to SPM (Fig. 4c), pointing to enzymatic dysfunction. Consistent with these findings, BODIPY-SPD/SPM uptake activity of cells overexpressing KRS mutants (T512I and G872R) was lower than that of WT cells, whereas cells expressing EOPD mutants (T12M, G528R and A741T) displayed an intermediate phenotype (Fig. 4d-e). Together, all tested disease mutations disturb ATP13A2-dependent polyamine transport, and the degree of functional impact correlates with the EOPD *versus* KRS disease phenotype.

The present invention demonstrates that loss of ATP13A2 exacerbates polyamine-induced toxicity
Defective ATP13A2 causes general lysosomal dysfunction¹⁴ that is central in the disease mechanism⁴⁴. Here, we investigated whether defective lysosomal polyamine export explains the lysosomal phenotype in ATP13A2 KO cells¹⁴.
SPM and SPD are highly abundant and physiologically important organic polycations that support protein, membrane and DNA stability⁴⁵. However, at high concentrations, polyamines become toxic to cells⁴⁵, and we explored here whether toxicity arises due to lysosomal dysfunction. We first confirmed that increasing concentrations of SPM or SPD (for 24 h) reduced the viability of control cells (Fig. 14c-d), which was paralleled by an increase in cell death (Fig. 5a), whereas ornithine and putrescine were not cytotoxic (Fig. 14a-b). Importantly, comparisons of KO, KO/WT and KO/D508N *versus* control cells clearly showed that ATP13A2 activity reduces SPM/SPD toxicity (Fig. 5a, Fig. 14c-d), pointing to a lysosomal role in this phenotype. Since polyamines are not lysosomotropic⁴⁶, we hypothesized that SPM enters KO or KO/D508N cells via endocytosis (Fig. 2f), but becomes trapped due to impaired lysosomal SPM export (Fig. 2g-j), which may lead to lysosomal dysfunction.
We therefore investigated the effects of SPM on lysosomal functionality at a time point preceding cell death (4 h, Fig. 14e) to ensure we were observing lysosomal dysfunction as a consequence of polyamine exposure and not due to the secondary effects of cell death. First, we demonstrated that lysosomal acidification was compromised in KO and KO/D508N cells (Fig. 5b). At a SPM concentration that induced toxicity in an ATP13A2-dependent manner (10 µM, Fig. 5a), lysosomal pH further increased in the KO and KO/D508N cells (Fig. 5b). Moreover, this neutralizing effect was absent in control and KO/WT cells (Fig. 5b). Lysosomal alkalization may explain the diminished lysosomal degradation potential observed in KO and KO/D508N cells (Fig. 5c). At toxic SPM levels, we observed decreased cathepsin D (CTSD) activity (Fig. 14g) and increased cathepsin B (CTSB) activity (Fig. 5d), which may relate to differences in their pH optima. These findings may also point to loss of lysosomal membrane integrity⁴⁷, a driver of lysosome-dependent cell death⁴⁸ and a mediator of neuronal death⁴⁹. Utilizing an acridine orange (AO)-based assay designed to assess membrane integrity⁵⁰, we showed that ATP13A2 KO and KO/D508N cells demonstrated impaired lysosomal membrane integrity (Fig. 5e). KO and KO/D508N cells presented with stronger AO staining, which was already significant under basal conditions, but became more prominent following SPM challenge, a phenotype that was absent in CON and KO/WT cells (Fig. 5e). Moreover, no galectin 3 (Gal-3) punctae were observed in CON or KO/WT cells, whereas SPM treatment increased both the number and size of endogenous Gal-3 punctae in KO and KO/D508N cells, which is indicative for lysosomal rupture (Fig. 5f). Lysosomal rupture was further confirmed by demonstrating the loss of endo-lysosomal FITC-dextran punctae and a more diffuse, cytosolic CTSB staining only in the KO and KO/D508N cells (Fig. 14h-i). Altogether, these results point toward SPM-induced lysosomal rupture following ATP13A2 deficiency⁵¹.
Loss of ATP13A2 leads to lysosomal dysfunction, which can be rescued by administration of acidic nanoparticles⁴⁴. Here, we evaluated whether acidic nanoparticles also prevent the increased SPM-mediated toxicity in ATP13A2 KO cells. Exposure to acidic nanoparticles restored lysosomal pH and functionality (Fig. 5b-c), prevented CTSB activation (Fig. 5d) and recovered lysosomal membrane intactness (Fig. 5e) ultimately reducing SPM-induced cell death (Fig. 5g). This shows that lysosomal dysfunction appears as an early and critical defect in the polyamine toxicity phenotype. Furthermore, we found that CTSB inhibition significantly reduced the toxic effect of SPM in the KO and KO/D508N cells (Fig. 5h), demonstrating that activation and lysosomal release of CTSB contributes to the elevated SPM cytotoxicity.
We conclude that loss of ATP13A2 leads to SPM-induced lysosomal membrane rupture, causing cytotoxicity and lysosome-dependent cell death via CTSB⁴⁸, providing a likely mechanism for the increased SPM-induced toxicity in ATP13A2 KO cells.
The present invention demonstrates that loss of ATP13A2 orthologues sensitizes primary neurons and nematodes to polyamine toxicity
Lysosomal polyamine toxicity may be relevant in the context of neurodegeneration, since isolated mouse cortical neurons with miRNA-mediated Atp13a2 knockdown (Fig. 6a) were more susceptible to cell death following exposure to SPM than control neurons (miR-Fluc, miRNA targeted to Fluc) (Fig. 6b). Interestingly, increased SPM sensitivity of neurons with Atp13a2 knockdown can be rescued either by inhibition of CTSB activity (Fig. 15); or by lentiviral transduction of human ATP13A2 WT, but not the catalytically dead D508N mutant (Fig. 6b).
Finally, polyamine toxicity also arises *in vivo* in *Caenorhabditis elegans* strains with deficiency in ATP13A2 orthologues (CATP-5-7⁵²). CATP-6 and CATP-7 show vesicular expression similar to ATP13A2, whereas CATP-5 is plasma membrane localized⁵². It has been previously reported that *catp-5(0)* worms are resistant to norspermidine (a toxic polyamine)²⁴. Since neither norspermidine nor SPM are produced in *C*. *elegans,* we assessed SPD toxicity in the three strains *catp-5(0), catp-6(0)* and *catp-7(0)* in comparison to N2 (wild-type). In SPD conditions, N2 control worms displayed hampered growth capacity, presented by developmental delay and, hence, reduced worm length. This phenotype was exacerbated in *catp-5(0), catp-6(0)* or *catp-7(0)* animals, which also appeared unhealthier (Fig. 6c). The phenotype of CATP-6- and CATP-7-deficient strains was rescued by expression of CATP-6 or CATP-7 WT, but not a catalytically dead variant, showing that SPD-induced toxicity depends on the lack of CATP-6 or CATP-7 transport activity, respectively (Fig. 6c).
Overall, we demonstrate that the loss of ATP13A2 orthologues sensitizes primary neurons and nematodes to polyamine toxicity.

ATP13A2 fulfils a novel lysosomal function operating as a late endo-lysosomal polyamine exporter that contributes to cellular polyamine uptake and protects lysosomal homeostasis. Polyamines exist in the extracellular environment in the micromolar range⁵³, are able to bind to plasma membrane heparan sulphate proteoglycans⁵⁴ prior to entering the cell via endocytosis³⁴ and are subsequently transported into the cytosol by ATP13A2. Our findings further suggest that this polyamine uptake route complements cytosolic polyamine synthesis via the ODC pathway (Fig. 6d). As such, ATP13A2 may be part of the elusive polyamine transport system in mammalian cells⁵⁵, supporting polyamine delivery to enhance cellular vitality. Also the other P5B-ATPases ATP13A3-5 reside in the endosomal system and share high sequence similarity in the substrate binding region³², and may together with ATP13A2 belong to the mammalian polyamine transport system.
ATP13A2 removes polyamines from lysosomes to maintain lysosomal pH, degradative capacity and membrane integrity. ATP13A2 KO cells manifest a basal increased lysosomal pH and reduced lysosomal membrane integrity, which are exacerbated under polyamine load as a consequence of lysosomal polyamine export deficiency leading to lysosome-dependent cell death via CTSB release and activation. Polyamine toxicity therefore represents a novel pathway of lysosome-dependent cell death that may contribute to neuronal loss when ATP13A2 is disturbed, suggesting that this may play a role in ATP13A2-associated neurodegeneration. Disturbed lysosomal functionality is central in the disease mechanism following ATP13A2 deficiency¹⁴, since treatment with acidic nanoparticles restored lysosomal acidification⁴⁴. In addition, defective ATP13A2 also diminishes cellular polyamine availability, which may further potentiate the disease phenotype. Indeed, polyamines such as SPM are important scavengers of reactive oxygen species⁵⁶, and heavy metals⁵⁷, and they also play a role in autophagy^{58,59}. The dual impact of ATP13A2 on both lysosomal and cytosolic polyamine levels may explain the broad phenotype associated with ATP13A2 loss of function^{4-7,43}.
Polyamines are vitally important polycations that are tightly regulated by a complex interplay of import, export, synthesis and degradation pathways⁶⁰. Polyamine levels decline with age⁶¹, whereas polyamine supplementation increases life span in several model organisms^{62,63}. Conversely, loss-of-function mutations in SPM synthase cause Snyder-Robinson syndrome, a form of X-linked intellectual disability⁶⁴, and reduced activity of the catabolic polyamine enzyme SPD/SPM N1-acetyltransferase 1 (SAT1) has been implicated in PD⁶⁵. Furthermore, lysosomal dysfunction arises due to mutations in a plethora of PD related genes, such as the lysosomal enzyme glucosylceramidase (GBA1, the most common PD risk gene)⁶⁶. It remains to be studied whether other PD genes may affect ATP13A2 functionality and impact on lysosomal polyamine export or sensitivity, which may contribute to neurodegeneration.
Here, we establish KRS as a novel polyamine-associated neurological disorder and our findings suggest that a disturbed polyamine homeostasis is also implicated in other ATP13A2-associated diseases, such as EOPD, NCL, HSP and ALS. Stimulating lysosomal polyamine export may therefore be a strategy for neuroprotective therapy. Interestingly, the functional impact of KRS and EOPD disease mutations in ATP13A2 correlates well with the disease phenotype. Note however that ATP13A2 truncation mutations were also reported in HSP⁷ and NCL⁶⁷, and the T512I substitution can cause either HSP or KRS, indicating that the mutation type is not the sole determinant of the clinical phenotype⁷. Mis-localization of ATP13A2 disease mutants can also contribute to the disease phenotype^{7,68}.
In conclusion, ATP13A2 exports polyamines from the late endo-lysosomal compartment, which promotes lysosomal health and functionality. Conversely, ATP13A2 deficiency disturbs lysosomal activity and integrity thereby sensitizing cells to toxic effects of exogenous polyamines. This may explain why defects in ATP13A2 lead to neurodegeneration.

### EXAMPLE 1: materials and methods

**Materials** - The following reagents were purchased from Sigma-Aldrich: sodium orthovanadate (S6508), CaCl₂ (C3881), ZnCl₂ (Z0152), MnCl₂ (M3634), FeCl₃ (157740), SPM (S3256), SPD (S2626), N¹-acetylspermine trihydrochloride (01467), N¹-acetylspermidine hydrochloride (9001535-1), N⁸-acetylspermidine dihydrochloride (A3658), putrescine dihydrochloride (P7505), L-arginine (A5006), L-ornithine monohydrochloride (O2375), histamine (H7250), agmatine sulfate salt (A7127), dopamine hydrochloride (H8502), cadaverine (D22606), yeast nitrogen base without amino acids (Y0626), yeast drop-out mix without uracil (Y1501), glucose (G8720), streptavidin sepharose (GE17-5113-01), thrombin (GE27-0846-01), DMSO (276855), DFMO (D193), berenil (D7770), Dynasore (D7693), Pitstop 2 (SML1169), 4-methylumbelliferyl heptanoate (MUH, M2514), PI (P4170), CA-074 (C5732), fluorescein isothiocyanate-dextran (FITC-dextran, 46945), DAPI (D9542), anti-ATP13A2 antibody (A3361), anti-GAPDH antibody (G8795), Resomer® RG 503H (719870) and SigmaFast protease inhibitor (S8820). In addition, 18:1 PI(3,5)P2 [1,2-dioleoyl-sn-glycero-3-phospho-(1'-myo-inositol-3',5'-bisphosphate) (ammonium salt); 850154], 18:1 PA [1,2-dioleoyl-sn-glycero-3-phosphate (sodium salt); 840875] and egg phosphatidylcholine (840051) were obtained from Avanti Polar Lipids. Bovine serum albumin (BSA; 3854.3) was obtained from Carl Roth. Yeast extract (103753.0500) was purchased from VWR, and n-dodecyl-β-D-maltopyranoside (DDM; 1758-1350) was purchased from Inalco. We obtained Bio-Beads SM-2 resin (1523920) from Bio-Rad. ³H-SPM (ART 0471) was ordered from ARC. APCHA (sc-202715) and 4MCHA (sc-272662) were purchased from Santa Cruz Biotechnology. Genistein (ab120112), anti-galectin-3 antibody (ab2785), anti-LAMP1 antibody (Abcam, ab24170) and anti-cathepsin B antibody (ab58802) were purchased from Abcam. TRYPLE (12604021), AO (A1372) and DQ-Green BSA (D12050) were ordered from Life Technologies.

**Compound/inhibitor preparation** - All polyamines, diamines, monoamines and amino acids were prepared to a final stock concentration of 500 mM (200 mM in the case of SPM) in 0.1 M MOPS-KOH (pH 7.0). DFMO was prepared to a final stock concentration of 500 mM in mQ H₂O. The inhibitors 4MCHA and APCHA were dissolved in DMSO to a final stock concentration of 200 mM. The endocytosis inhibitors Dynasore, genistein and Pitstop 2 were dissolved in DMSO to final concentrations of 50 mM, 25 mM and 25 mM, respectively.

**Generation of SH-SY5Y cell models** - SH-SY5Y human neuroblastoma cells were transduced with lentiviral vectors to obtain stable overexpression of firefly luciferase (Fluc) or ATP13A2 (isoform 2, WT, indicated disease or catalytic mutants) and maintained as described previously [Holemans, T. et al. Proc Natl Acad Sci U S A 112, 9040-9045, (2015); Martin, S. et al. Cell Cycle 14, 3341-3342, (2015); Martin, S. et al. Parkinson's disease 2016, 9531917, (2016)]

For CRISPR/Cas9-mediated KO of ATP13A2, the lentiviral vector lentiCRISPRv2 (Addgene: #52961) was used [Sanjana, N. et al. Nat Methods 11, 783-784, (2014)]. First, the Cas9 cassette was transformed into a high-fidelity Cas9 (Cas9-HF) by Gibson assembly with a gBlock gene fragment (Integrated DNA Technologies) of the Cas9 portion encoding a protein product with HF mutations (N497A/R661A/Q695A/Q926A). This Cas9 variant triggers less off-target events while retaining its on-target activity [Kleinstiver, B. P. et al. Nature 529, 490-495, (2016)]. A single-guide RNA (sgRNA) targeted to Atp13a2 was designed taking into account a high on-target efficiency, using sgRNA Designer [Doench, J. G. et al.. Nat Biotechnol 34, 184-191, (2016)], and a low off-target efficiency, via CRISPR Design (http://crispr.mit.edu/). A lentiviral CRISPR/Cas9 HF expression plasmid was created by inserting fragments that contained an sgRNA sequence of ATP13A2 into the lentiCRISPRv2 vector. The generated CRISPR-Cas9 HF-ATP13A2 plasmid (1,000 ng), the packaging plasmid pCMV-ΔR8.91 (900 ng), and the envelope plasmid pMD2G-VSV-G (Addgene, 12259) (100 ng) were mixed together with 200 µl of JetPrime buffer and 4 µl of JetPrime reagent (Polyplus-transfection) for transfection of HEK-293T cells according to the manufacturer's protocol. After 4 hours (h) at 37°C and 5% CO₂, the serum-free medium was replaced with DMEM/F12 (Dulbecco's modified Eagle's medium, Nutrient Mixture F-12) supplemented with 10% fetal calf serum (heat-inactivated). 48 h later, the lentiviruses were collected by passing the medium through a 0.45 µm filter, and 0.5 ml of this medium was used to transduce SH-SY5Y cells supplemented with 8 µg/ml polybrene (Sigma-Aldrich). 24 h after transduction, the infected cells were selected in 3 µg/ml puromycin (Sigma-Aldrich) and passaged three times before single clones were isolated via serial dilution. The resulting cells were examined by qPCR and Western blotting. For rescue experiments, ATP13A2 KO cells were stably transduced with lentiviral vectors expressing ATP13A2 WT or D508N mutant in which both cDNAs were modified with synonymous mutations at the sgRNA target site.

**Membrane fractionation** - Transduced SH-SY5Y cells were seeded in 15 cm² dishes at a density of 6 x 10⁶ cells per plate. 24 h later, the cells were harvested following trypsinization and brief centrifugation (300 x g, 5 min). Subcellular fractionation was performed by differential centrifugation. The microsomal protein concentration was measured using the bicinchoninic acid assay (Thermo Fischer Scientific, Pierce) according to the manufacturer's instructions.

**ATPase assay** - The ATPase activity of ATP13A2 was assessed using a commercially available luminescence assay (ADP-Glo Max assay, Promega) that monitors the production of ADP via luciferase activity. The reactions were performed for 30 min (37°C) in a final volume of 25 µl. The assay reaction mixture contained 50 mM MOPS-KOH (pH 7), 100 mM KCI, 11 mM MgCl₂, 1 mM DTT, 195 µM DDM, various concentrations of the indicated compound, and either microsomes (5 µg) harvested from SH-SY5Y cells overexpressing ATP13A2 (WT or mutants) or purified ATP13A2 (0.5 µg). When purified ATP13A2 was used, we included 125 µM PA, 125 µM PI(3,5)P₂ and 19.5 µM DDM in the reaction buffer. The assay was started by the addition of 5 mM ATP and was terminated by adding 25 µl of ADP-Glo Reagent. The 96-well plate was then incubated for 40 min at room temperature, followed by the addition of 50 µl of ADP-Glo Max Detection Reagent. After 40 min, luminescence was detected using a FlexStation 3.0 system (Molecular Devices). Dose-response curves and n/Kₘ/Vₘₐₓ values were calculated using GraphPad Prism Software (GraphPad Software Inc.).

**Auto-phosphorylation assay** - The auto-phosphorylation activity of ATP13A2 on the conserved aspartate D508 was measured as described previously [Holemans et al. cited above; Estrada-Cuzcano, A. et al. Brain 140, 287-305, (2017); Sorensen, D. M. et al.. PLoS One 13, e0193228, (2018)].

Briefly, microsomes (20 µg) or purified ATP13A2 (1 µg) were incubated with radioactively labelled ATP in the presence of the indicated SPM or ornithine concentrations, and after 1 min, the reaction was stopped. In case of purified ATP13A2, 125 µM PA and 125 µM PI(3,5)P2 were included in the reaction mixture. To determine the sensitivity of the ATP13A2 phosphoenzyme to ATP or a combination of ATP and SPM, 30 s after adding ³²P-ATP, samples were incubated with non-radioactive ATP (5 mM in experiments using microsomes, 1 mM when purified ATP13A2 was used) and SPM (1 mM) before the reaction was stopped at indicated time points. The incorporation of ³²P was visualized following SDS-PAGE under acidic conditions and subsequently detected by autoradiography (microsomes) or liquid scintillation counting (purified ATP13A2) (Liquid Scintillation Analyzer TRI-CARB 2900TR).

**Transformation and overexpression of ATP13A2 in yeast** - The *S*. *cerevisiae* W303-1B/Gal4-ΔPep4 strain (leu2-3, his3-11,15, trp1-1::TRP1-GAL10-GAL4, ura3-1, ade2-1, canr, cir⁺, ΔPep4 MAT; a kind gift from Rosa Lopez Marques) was transformed according to the lithium acetate/single-stranded carrier DNA/polyethylene glycol method [Gietz, R. D. & Woods, R. A. Methods Enzymol 350, 87-96 (2002)], with the pYeDP60 vector containing a yeast codon optimized version of human *ATP13A2* variant 2 (WT or the catalytically dead E343A mutant) followed by a thrombin cleavage site and a C-terminal BAD tag [Azouaoui, H. et al. PLoS One 9, e112176, (2014); Jidenko, M. et al.Protein Expr Purif 48, 32-42, (2006)]. The transformation mixture was grown for 48 h at 30°C on minimal medium agar plates lacking uracil (0.54% yeast nitrogen base without amino acids, 0.12% yeast drop-out mix without Ura, 2% glucose, and 2% agar) to select yeast colonies that acquired the plasmid. These colonies were then cultured in 20 ml of MM-Ura medium (0.67% yeast nitrogen base without amino acids, 0.19% yeast drop-out mix without Ura, and 2% glucose) and grown for 24 h at 28°C and 200 rpm. The MM-Ura yeast pre-culture was used to inoculate 100 ml of MM-Ura medium to a final OD₆₀₀ of 0.2, followed by a 12 h incubation period (28°C and 200 rpm). The second pre-culture was inoculated into 4.5 l of YPGE2X medium (2% yeast extract, 2% bactopeptone, 1% glucose, and 2.7% ethanol) to a final OD₆₀₀ of 0.05 and grown for 36 h (28°C and 175 rpm). ATP13A2-BAD expression was induced with 2% galactose, followed by a second galactose induction 12 h later. After another 12 h, the pellet was collected (1,000 x g; 10 min; 4°C).

**Yeast membrane preparation** - Yeast cells were broken with glass beads using a BeadBeater (BioSpec products). The lysis buffer contained 50 mM Tris-HCI (pH 7.5), 1 mM EDTA, 0.6 M sorbitol, 1 mM phenylmethylsulfonyl fluoride and SigmaFast protease inhibitor. To remove cell debris and nuclei, the crude extract was centrifuged at 2,000 x g for 20 min (4°C). The supernatant (S1) was centrifuged at 20,000 x g for 20 min (4°C) to pellet the heavy membrane fraction (P2), and the resulting supernatant (S2) was further centrifuged at 200,000 x g for 1 h (4°C). The resulting pellet (*i.e.,* the light membrane fraction, P3) was resuspended in 20 mM HEPES-Tris (pH 7.4), 0.3 M sucrose and 0.1 mM CaCl₂. The total protein concentration was determined using a Bradford assay (B6916, Sigma-Aldrich).

**ATP13A2-BAD purification** - The method used to purify ATP13A2 was based on the purification of overexpressed Drs2p from yeast membranes. Yeast P3 membranes were diluted to 5 mg of total protein/ml in SSR buffer (50 mM MOPS-KOH (pH 7), 100 mM KCI, 20% glycerol, 5 mM MgCl₂, 1 mM DTT, and SigmaFast protease inhibitor cocktail) and solubilized using DDM, with a detergent to protein ratio of 1:1. The samples were stirred on ice for 30 min, followed by centrifugation (100,000 x g; 1 h; 4°C) to pellet non-solubilized membranes. The solubilized material was incubated with streptavidin beads for 4 h (4°C) to allow binding of BAD-tagged ATP13A2 to the resin. To eliminate unbound material, the resin was washed four times with three resin volumes of SSR buffer supplemented with 0.5 mg/ml DDM. Subsequent cleavage by thrombin (0.0625 U/mg total protein) allowed the release of ATP13A2 from the beads by overnight incubation at 4°C. Finally, a Vivaspin Turbo 4 concentrator (100 kDa MWCO, Sartorius) was used to concentrate the sample. The protein concentration was determined using a Bradford assay. The quality of the purification was evaluated via SDS-PAGE followed by Coomassie staining or immunoblotting. Furthermore, the purified ATP13A2 sample was analyzed by linear mode MALDI-TOF MS (Applied Biosystems 4800 Proteomics Analyzer) in the presence of α-Cyano-4-hydroxycinnamic acid as matrix and after C4 omix (Agilent) pipette tip purification.

**Reconstitution of yeast membranes** - To reconstitute yeast membranes, we followed a similar strategy as described before [Papadopulos, A. et al. J Biol Chem 282, 15559-15568, (2007)].

P3 membranes from the yeast membrane preparation expressing the ATP13A2-BAD construct were solubilized in buffer T (10 mM Tris-HCI (pH 7.4) and 1 mM EDTA) supplemented with 1.4% DDM. After removing the insoluble fraction by ultracentrifugation (30 min; 200,000 x g), the detergent extract was supplemented with 4.5 mM egg phosphatidylcholine and 0.5 mM 18:1 PA (in buffer T containing 0.7% DDM). The extract was then treated with Bio-Beads to remove the DDM and reconstitute proteoliposomes (*i.e.,* "no ATP inside" condition). To generate proteoliposomes that contained intraluminal ATP (*i.e.,* "ATP inside" condition), we added 5 mM ATP and an ATP-regenerating system before incubation with the Bio-Beads. Finally, the vesicles were recovered by ultracentrifugation (1 h; 200,000 x g) and resuspended in buffer T. The protein concentration was determined using a Bradford assay.

**Transport assay using reconstituted vesicles** - ³H-SPM uptake into freshly prepared vesicles was measured within 60 min. The above-described vesicles ("no ATP inside" or "ATP inside") were diluted to 1 µg/µl in buffer T. The reactions were performed for 10 min (30°C) in a final volume of 1 ml. The assay reaction mixture contained 50 mM MOPS, 100 mM KCI, 11 mM MgCl₂, 1 mM DTT and reconstituted vesicles (45 µg). The reaction was started by adding 1 mM ³H-SPM. For the condition "no ATP inside", 5 mM ATP and an ATP-regenerating system were added after Bio-Bead treatment, prior to the addition of ³H-SPM. The reaction was stopped by filtering the samples through Millipore filters (0.45 µm). Following washing of the filters with assay buffer, radioactivity retained on the filters was counted using a liquid scintillation counter (Liquid Scintillation Analyzer TRI-CARB 2900TR).

**Cellular transport assay and endocytosis assessment** - BODIPY-SPD and BODIPY-SPM were synthesized as described previously (compounds 14 and 15, respectively) [Vanhoutte, R. et al.. Chembiochem 19, 907-911, (2018)].

The cells were seeded in 12-well plates (1.0 X 10⁵ cells per well) and the next day, the cells were incubated with 5 µM BODIPY-SPM or BODIPY-SPD for 2 h before harvesting. To assess endocytosis, the cells were pre-treated (30 min) with the endocytosis inhibitors Dynasore (100 µM), genistein (50 µM) and/or Pitstop 2 (50 µM) prior to the addition of either 20 µg/ml FITC-dextran or BODIPY-SPM (2 h and 37°C). The cells were then collected, washed and resuspended in PBS containing 1% BSA. Finally, an Attune Nxt (Thermo Fischer Scientific) flow cytometer was used to measure the mean fluorescence intensities (MFI) of 10,000 events per treatment.

**Metabolomics** - Cells were grown into a 6-well and extracted as described in Byun, J. A. et al.. Biomed Chromatogr 22, 73-80 (2008). Briefly, the medium was removed and cells were washed with a 0.9% NaCl solution. The washing solution was removed and 150 µl of a 6% trichloroacetic acid (TCA, Sigma) was added for the extraction. Using a cell scraper, the full extract was transferred into an eppendorf and incubated for 30 min on ice. Insolubilities such as precipitated proteins were removed by centrifugation for 20 min at 20,000 x g at 4°C. To 100 µl of the supernatant, 900 µl of a 100 mM sodium carbonate buffer (pH 9.0) was added. Next, 25 µl of isobutyl chloroformate (Sigma) was added and the mixture was incubated for 30 min at 35°C. 800 µl of the reaction mixture was transferred to a 2 ml eppendorf tube and 1 ml of diethylether (Sigma) was added. The mixture was vortexed vigorously and placed for 15 min at 25°C. 900 µl of the upper phase was transferred into an eppendorf and dried using a vacuum centrifuge. Finally, the dried extract was dissolved in 125 µl of a 50% acetonitrile (LC-MS grade, Merck) solution in water containing 0.2% acetic acid. Medium samples were extracted by adding 100 µl of 4% TCA to 100 µl of medium. The subsequent modifications were similar as described above.

15 µl of the extract was loaded onto a Thermo Scientific Liquid Chromatography QQQ (Quantiva, Thermo Scientific) equipped with an ACQUITY UPLC BEH C18 (1.7 µm, 2.1 x 100 mm) column from WATERS. Solvent A consisted of mQ H₂O with 0.2% acetic acid while solvent B was acetonitrile (Merck) with 0.2% acetic acid; all solvents used were LC-MS grade. Flow rate remained constant at 250 µl/min, and the column temperature remained constant at 30°C. A gradient for the separation of modified polyamines was applied as follows: from 0 to 2 min 20% B, from 2 to 10 min a linear increase to 85% B was carried out and remained at 85% B until 17 min. At 18 min the gradient returned to 20% B. The method stopped at 22 min. The MS operated in positive ion mode (3500 V); the source settings were as follows: Sheath gas at 50, Aux Gas at 10. Ion Transfer tube was heated at 325°C and Vaporizer temperature was set at 350°C. The MS operated in MRM mode and used the following transitions: ornithine (parent m/z at 333.2 - -> fragment 315.2, collision energy at 10.25V), putrescine (parent m/z at 289.2 --> fragment 215.2, collision energy at 10.25V), SPD (parent m/z at 446.4 --> fragment 198.2, collision energy at 23.6V) and SPM (parent m/z at 603.4 --> fragment 455.1, collision energy at 19.91V). Peak area was integrated using the XCalibur Quan tool (version 4.2.28.14, Thermo Scientific).

**Preparation and characterization of acidic nanoparticles** - The nanoparticles were prepared according to Bourdenx, M. et al. Autophagy 12, 472-483, (2016). Briefly, 31 mg of Resomer® RG 503H (lactide to glycolide ratio 50:50, molecular weight 24-38 kDa) were dissolved in 3.1 ml of THF and subsequently 200 µl of this solution were added to 20 ml of ultrapure water under sonication. The suspension was then concentrated at the rotary evaporator to about 12 ml, resulting in a concentration of 0.167 mg/ml. The size distribution of prepared nanoparticles was measured using a Wyatt DynaPro DLS platereader (Wyatt, Santa Barbara, USA), employing an 830 nm laser in a flat-bottom 384-well plate (Greiner, Frickenhausen, Germany) at 25 °C and 10 measurements were averaged.

**MUH cytotoxicity assay** - SH-SY5Y cells were seeded in 96-well plates (1 X 10⁴ cells per well) and allowed to adhere overnight. The cells were subsequently treated with increasing doses of the indicated compounds for 24-48 h. Following exposure, the cells were washed with PBS and stained with 300 µg/ml MUH (prepared in DMSO, dissolved in PBS) for 30 min at 37°C. Cytotoxicity was read using a FlexStation 3.0 multi-well plate reader (Molecular Devices; excitation 360 nm, emission 460 nm, and cut-off 455 nm).

**PI exclusion assay** - Cells were seeded in 12-well plates (1.0 X 10⁵ cells per well) and the next day, the cells were treated with increasing doses of SPM, alone or in combination with the CTSB inhibitor CA-074 (25 µM, 1 h pre-incubation) or acidic nanoparticles (180 ng/ml, 1 h pre-incubation), and incubated for 24 h at 37°C. Thereafter, the cells were harvested following trypsinization and a brief centrifugation (300 x g; 5 min), washed with PBS and stained with 1 µg/ml PI (in PBS containing 1% BSA). An Attune Nxt (Thermo Fischer Scientific) flow cytometer was used to determine the proportion of PI-positive cells (10,000 events per treatment).

**FITC-dextran based lysosomal pH** - SH-SY5Y cells were seeded in 12-well plates (1.0 X 10⁵ cells per well) and allowed to adhere overnight. Cells were exposed to 50 µg/ml FITC-dextran for 72 h. Samples were then washed, placed in fresh media and treated with SPM (10 µM) alone or in combination with acidic nanoparticles (180 ng/ml, 1 h pre-incubation) for 4 h. Samples were then collected by centrifugation (450 x g, 5 min) and washed in PBS. Cells were finally resuspended in 500 µl of PBS containing 1% BSA and FITC dual emission assessed by flow cytometry (excitation 488 nm, emission 530 (BL1) and 600 (BL2)) using an Attune NXT flow cytometer (Invitrogen). The emission (BL1/BL2) of all samples were compared to a standard curve, whereby signals were obtained from control cells resuspended in monensin (100 µM) containing Britton Robinson buffer with increasing pH (3.0-8.0).

**Lysosomal degradative capacity** - Cell lines were seeded in 12-well plates (1.0 X 10⁵ cells per well) and the next day, the cells were pre-treated with SPM (10 µM) for 1 h at 37°C. For samples requiring acidic nanoparticle exposure, cells were treated with 180 ng/ml 1 h prior to SPM addition. Subsequently, 5 µg/ml DQ-Green BSA was added to the cells for a further 3 h (37°C). Finally, the cells were collected, and the MFI of 10,000 events were assessed using an Attune Nxt (Thermo Fischer Scientific) flow cytometer.

**Cathepsin activity assays** - SH-SY5Y cells were seeded in 10 cm² plates (2 X 10⁶ cells per plate) and allowed to adhere overnight prior to treatment with 10 µM SPM for 4 h at 37°C. For samples requiring acidic nanoparticle addition, 180 ng/ml nanoparticles were added 1 h prior to SPM addition. Next, the samples were harvested using TRYPLE and a brief centrifugation (300 x g; 5 min). CTSB (ab65300) and CTSD (ab65302) activities were assessed using commercially available kits (Abcam) according to the manufacturer's instructions. MFI were acquired using a FlexStation 3.0 multi-well plate reader (Molecular Devices).

**Lysosomal membrane integrity** - SH-SY5Y cells were seeded in 12-well plates (1.0 X 10⁵ cells per well) and the next day, the cells were incubated with 5 µg/ml AO (dissolved in media) for 15 min at 37°C. Thereafter, the medium was discarded, the cells were washed, and fresh medium was added. For samples requiring acidic nanoparticles, cells were treated with 180 ng/ml 1 h prior to SPM addition. The cells were then treated with 10 µM SPM for 4 h at 37°C. Finally, the cells were collected and resuspended in PBS containing 1% BSA. The MFI of 10,000 events was captured using an Attune Nxt (Thermo Fischer Scientific) flow cytometer.

**BODIPY-SPM localization and lysosomal rupture analysis** - For all immunofluorescent stainings SH-SY5Y cell lines were seeded in 12-well plates (0.25 X 10⁵ cells per well on coverslips) and the next day, the cells were incubated with the indicated compounds for 4 h at 37°C. For BODIPY-SPM analysis, cells were pulsed with 5 µM SPM for 15 min, washed and chased in fresh media for a further 1 h and 45 min at 37°C. Following treatments, the cells were washed twice in PBS, fixed in 4% paraformaldehyde for 30 min (37°C), washed in PBS and stored at 4°C. For immunofluorescence staining, the cells were washed in PBS containing 0.5% Tween 20 (PBS-T), permeabilized in PBS containing 0.1% Triton X-100 (30 min) and blocked first in 0.1 M glycine and then in PBS-T containing 1% fetal calf serum and 10% BSA. Gal-3-, LAMP1- and cathepsin B-specific antibodies were used at a dilution of 1:100-200 (in PBS-T containing 1% BSA) overnight at 4°C. Subsequently, the samples were washed and incubated with Alexa Fluor™ secondary antibody (1:1,000, 30 min). To assess FITC-dextran release, cells were loaded with 50 µg/ml FITC-dextran for 72 h, cells were then washed and placed in fresh media for 1 h prior to SPM (10 µM) addition. To visualize the nucleus, all samples were stained with DAPI. Following staining, the samples were fixed to the slides, and images were acquired using an LSM780 or LSM880 confocal microscope (Zeiss). For the acquisition of BODIPY-SPM, images were taken with equal settings to confirm uptake potential of ATP13A2 (Fig. 2g). To assess the intracellular distribution of BODIPY-SPM (Fig. 2h-k), microscope settings were optimized per cell type to allow a comparable assessment of BODIPY-SPM localization in KO/WT and KO/D508N cells.

**Neuron isolation** - Primary cortical neurons were derived from E16 FVB/N mice embryos. Pregnant mice were sacrificed on gestation day 16 by cervical dislocation. E16 mice pup brains were collected and placed in a dish containing calcium- and magnesium-free Hanks' Balanced Salt Solution (HBSS, Life Technologies, 14180-046) on ice. Both cerebral hemispheres were separated from the cerebellum. Meninges were removed from the cerebral hemispheres and the brain cortices dissected. Brain cortices were collected, washed twice and digested with 0.05% trypsin (Life Technologies, 25300-054, 10 min at 37°C). Trypsin reaction was terminated by the addition of 7 ml HBSS and 1 ml of horse serum. Cells were separated by pipetting and filtration through a cell strainer (40 µm, Falcon, 352340). Cells were centrifuged at 1,000 rpm for 5 min (4°C), the supernatant discarded and the pellet suspended in 5 ml Dulbecco's Modified Eagle Medium (DMEM; Sigma-Aldrich, D6546) containing GlutaMAX (Life Technologies, 31966-021) containing 5% horse serum (Life Technologies, 26050-088) and 20 mM glucose (Sigma-Aldrich, 8270). Primary cortical neurons were plated in 12 well plates, on cover slips coated with poly-D-lysine (Sigma-Aldrich, P6407). After an overnight incubation, cell medium was exchanged for Neurobasal medium (Life Technologies, 21103-049) supplemented with 2 mM L-glutamine (Life Technologies, 25030-24) and 2% B27 (Life Technologies, 17504-044).

**Atp13a2 knockdown and rescue in isolated cortical neurons** - For knockdown, microRNA (miR) based short-hairpin lentiviral vectors were generated as described in Osorio, L. et al. Journal of biotechnology 169, 71-81, d (2014). The two most potent miR's against mouse Atp13a2, mouse miR-3 and mouse miR-5 were used to induce knockdown in mouse primary neurons. A miR targeting firefly luciferase (Fluc) was used as a control Primary neurons were transduced 4 days after isolation, 72 h prior to experimentation. For rescue of ATP13A2 expression in knockdown conditions, neurons were subjected to a second round of transductions with either human WT or D508N variants of ATP13A2 24 h post miR addition. Fluc was used as an overexpression control. At day 7 post-isolation, cells were treated with 10 µM SPM for 24h. To test the contribution of CTSB in SPM induced cell death, neurons were pre-treated (30 min) with 10 µM CA-074. Knockdown efficiency was validated with qPCR on mRNA levels 72h after transduction with Gapdh as internal control

**TUNEL staining** - TUNEL staining was assessed according to the manufacturer's protocol for the Click-iT Plus TUNEL assay (ThermoFisher, C10617). DAPI was used as a nuclear counterstain and images were acquired using a Leica LSM780 confocal microscope.

***C. elegans*** - During routine culture, nematodes were grown on NGM (nematode growth medium) using *E. coli* strain AMA1004 as food source [Brenner, S. Genetics 77, 71-94 (1974); Casadaban, M. J. et al. Methods Enzymol 100, 293-308 (1983)].

In order to reduce the likelihood that bacterial catabolism and divalent cations would interfere with SPD assays, these were done using DCDA (divalent cation depleted agar) plates, which do not permit bacterial growth. This medium contains; **a)** 2% agar (Carl Roth 5210) that has been washed with 50 mM EDTA, followed by multiple washes with reverse osmosis purified water, **b)** 50 mM HEPES pH 7.4 and **c)** 20 µg/ml kanamycin. Spermidine trihydrochloride (1 M stock solution; Sigma S2501) was added to a final concentration of 5 mM after microwaving to melt the agar. Assays were done in 35 mm plates that contained 1 ml of DCDA. Approximately 2 µl of *E. coli* was transferred to each assay plate from the lawn of a seeded NGM plate. In each case, multiple adult hermaphrodites were added to the assay plate and allowed to lay eggs for 8 h (data sets 1 and 3) or 15 h (data set 2) at 23.5°C, then removed. Incubation of plates at 23.5°C was continued until the 0 mM SPD N2 plates contained many mid-late stage L4s, at which point worms were rinsed off the plates and stored in microfuge tubes at - 20°C. Worm lengths were determined by mounting the thawed (dead) animals on an agarose pad covered by a coverslip, capturing images using a Leica M205 FA microscope equipped with digital camera plus software, and measuring the length of each worm from snout to tail tip using Image J.

The following mutant alleles were used, each of which was backcrossed to N2 Bristol at least three times: *catp-6(ok3473)* IV, *catp-7(tm4438)* IV, *catp-5(tm4481)* X [Consortium, C. e. D. MG3 (Bethesda) 2, 1415-1425, (2012)].

Each of these is a null allele, so they are referred to in the text as catp-#(0).

Transgenic strains carrying extrachromosomal arrays with the pRF4 *rol-6(su1006)* plasmid were generated by microinjection and maintained as described in Mello, C. C. et al. EMBO J 10, 3959-3970 (1991)]. Injection mixes typically contained 100 µg/ml pRF4, plus 30 µg/ml of the test construct. In some cases, Pmyo-2::gfp was included at approximately 5 µg/ml as an additional method for detecting transgenic animals. To generate transport-defective versions of *catp-6* and *catp-*7, plasmids carrying *catp-6::mKate2* and *catp-7::GFP*²¹ were modified to change the coding sequence for the conserved DKTGT autophosphorylation motif to NKTGT [Zielich, J. et al.. PLoS One 13, e0194451, (2018)].

Proper expression and subcellular localization of CATP-6::mKate2 and CATP-7::GFP was verified in the case of all transgenic strains that were used.

**Statistics** - Throughout this project, each experiment was performed a minimum of three independent times, and the data are expressed as the mean ± S.E.M. GraphPad Prism 7.0 was used to plot all graphs and to perform all of the required statistical and quantitative assessments. Statistical tests for each graph are described in the legend. For the cell biological experiments, unless directly specified, each cell model is the sum of two independent clones, each performed a minimum of three independent times.

### Cited References

1 Ross, C. A. & Poirier, M. A.. Nat Med 10 Suppl, S10-17, (2004).
2 Lee, J., Giordano, S. & Zhang, J. Biochem J 441, 523-540, (2012).
3 Klein, C. & Westenberger, A. Cold Spring Harb Perspect Med 2, a008888, (2012).
4 Ramirez, A. et al. Nature genetics 38, 1184-1191, (2006).
5 Di Fonzo, A. et al. Neurology 68, 1557-1562, (2007).
6 Bras, J., Verloes, A., Schneider, S. A., Mole, S. E. & Guerreiro, R. J. Hum Mol Genet 21, 2646-2650, (2012).
7 Estrada-Cuzcano, A. et al. Brain 140, 287-305, (2017).
8 Spataro, R. et al. Hum Genomics 13, 19, (2019).
9 Ramonet, D. et al. Hum Mol Genet 21, 1725-1743, (2012).
10 Gitler, A. D. et al. Nature genetics 41, 308-315 (2009).
11 Martin, S. et al. Parkinson's disease 2016, 9531917, (2016).
12 Holemans, T. et al. Proc Natl Acad Sci U S A 112, 9040-9045, (2015).
13 Martin, S., Holemans, T. & Vangheluwe, P.. Cell Cycle 14, 3341-3342, (2015).
14 Dehay, B. et al. Proc Natl Acad Sci U S A 109, 9611-9616, (2012).
15 Grunewald, A. et al. Neurobiol Aging 33, 1843 e1841-1847, (2012).
16 Palmgren, M. G. & Nissen, P. Annual review of biophysics 40, 243-266, (2011).
17 Kuhlbrandt, W. Nat Rev Mol Cell Biol 5, 282-295, (2004).
18 Axelsen, K. B. & Palmgren, M. G. Journal of molecular evolution 46, 84-101 (1998).
19 Covy, J. P., Waxman, E. A. & Giasson, B. I. J Neurosci Res 90, 2306-2316, 2 (2012).
20 Kong, S. M. et al. Hum Mol Genet 23, 2816-2833, (2014).
21 Narayanaswamy, N. et al. Nat Methods, (2018).
22 Lambie, E. J., Tieu, P. J., Lebedeva, N., Church, D. L. & Conradt, B. PLoS One 8, e77202, (2013).
23 De La Hera, D. P., Corradi, G. R., Adamo, H. P. & De Tezanos Pinto, F.. Biochem J 450, 47-53, (2013).
24 Heinick, A. et al. FASEB J 24, 206-217, (2010).
25 van Veen, S. et al. Frontiers in molecular neuroscience 7, 48, (2014).
26 Tan, J. et al.. J Biol Chem 286, 29654-29662, (2011).
27 Park, J. S., Koentjoro, B., Veivers, D., Mackay-Sim, A. & Sue, C. M. Hum Mol Genet 23, 2802-2815, (2014).
28 Rinaldi, D. E., Corradi, G. R., Cuesta, L. M., Adamo, H. P. & de Tezanos Pinto, F. Biochim Biophys Acta 1848, 1646-1655, (2015).
29 Yan, D. H. et al. J Physiol 563, 713-724, (2005).
30 Azouaoui, H. et al. A high-yield co-expression system for the purification of an intact Drs2p-Cdc50pPLoS One 9 (2014).
31 Andersen, J. P. et al. Frontiers in physiology 7, 275, (2016).
32 Sorensen, D. M. et al. PLoS One 13, e0193228, (2018).
33 Byun, J. A. et al. Biomed Chromatogr 22, 73-80 (2008).
34 Uemura, T., Stringer, D. E., Blohm-Mangone, K. A. & Gerner, E. W. Am J Physiol Gastrointest Liver Physiol 299, G517-522 (2010).
35 Vanhoutte, R., Kahler, J. P., Martin, S., van Veen, S. & Verhelst, S. H. L. System. Chembiochem 19, 907-911, (2018).
36 Demirsoy, S. et al. Hum Mol Genet 26, 1656-1669, (2017).
37 Tsunemi, T. et al. IJ Neurosci 39, 5760-5772, (2019).
38 Chatr-Aryamontri, A. et al. Nucleic Acids Res 45, D369-D379, (2017).
39 Funayama, M. et al.. Mov Disord 25, 2434-2437, (2010).
40 Lin, C. H. et al. Neurology 71, 1727-1732, (2008).
41 Mao, X. Y. et al. (2010). Parkinsonism Relat Disord 16, 235-236.
42 Usenovic, M., Tresse, E., Mazzulli, J. R., Taylor, J. P. & Krainc, D. J Neurosci 32, 4240-4246, (2012).
43 Santoro, L. et al. Neurogenetics 12, 33-39, (2011).
44 Bourdenx, M. et al. Autophagy 12, 472-483, (2016).
45 Pegg, A. E. J Biol Chem 291, 14904-14912, (2016).
46 Seglen, P. O. & Gordon, P. B.. Mol Pharmacol 18, 468-475 (1980).
47 Qiao, C. et al. Atp13a2 CNS Neurosci Ther 22, 451-460, (2016).
48 Aits, S. & Jaattela, M. Journal of cell science 126, 1905-1912 (2013).
49 Gan, L. et al. J Biol Chem 279, 5565-5572, (2004).
50 Lin, Y., Epstein, D. L. & Liton, P. B. Investigative ophthalmology & visual science 51, 6483-6495, (2010).
51 Aits, S. et al. Autophagy 11, 1408-1424, (2015).
52 Zielich, J. et al. PLoS One 13, e0194451, (2018).
53 Saiki, S. et al. Ann Neurol 86, 251-263 (2019).
54 Belting, M. et al. J Biol Chem 278, 47181-47189, (2003).
55 Poulin, R., Casero, R. A. & Soulet, D. Amino Acids 42, 711-723, (2012).
56 Ha, H. C. et al. Proc Natl Acad Sci U S A 95, 11140-11145 (1998).
57 Gaboriau, F. et al. Biochem Pharmacol 67, 1629-1637, (2004).
58 Eisenberg, T. et al.. Nat Cell Biol 11, 1305-1314, (2009).
59 Yang, Y. et al. I Cell Death Dis 8, e2738, (2017).
60 Pegg, A. E. IUBMB Life 61, 880-894, (2009).
61 Gupta, V. K. et al. Nat Neurosci 16, 1453-1460, (2013).
62 Eisenberg, T. et al. Nat Med 22, 1428-1438, (2016).
63 Madeo, F., Eisenberg, T., Pietrocola, F. & Kroemer, G.. Science 359, (2018).
64 Li, C. et al. SNat Commun 8, 1257, (2017).
65 Lewandowski, N. M. et al.. Proc Natl Acad Sci U S A 107, 16970-16975, (2010).
66 Sidransky, E. et al. The New England journal of medicine 361, 1651-1661, (2009).
67 Farias, F. H. et al. Neurobiol Dis 42, 468-474, (2011).
68 Podhajska, A. et al. PLoS One 7, e39942, (2012).
69 Sorensen, D. M., Buch-Pedersen, M. J. & Palmgren, M. G. Biochim Biophys Acta 1797, 846-855 (2010).

## Claims

1. An in vitro method of identifying compounds modulating (stimulating) polyamine transport into a cell comprising the step of:
a) providing a first eukaryotic cell culture wherein the endogenous P5B type ATPase gene is inactivated, wherein the cells comprise an inserted copy of a **wild type** or a **disease causing mutant** of the P5B type ATPase, and providing a second eukaryotic cell culture wherein the endogenous P5B type ATPase gene is inactivated, wherein the cells comprise an inserted copy of **an inactive** P5B type ATPase,
b) contacting the first and the second cell culture with a labelled polyamine and with a test compound,
c) measuring the uptake of the labelled polyamine by the first and the second cell culture,
d) determining the difference in uptake between the two cell cultures, wherein a difference in uptake between the first and the second culture, indicates that the test compound modulates polyamine transport.

2. The method according to claim 1, wherein the P5B type ATPase is ATP13A2.

3. The method according to claim 1 or 2, wherein the inactive ATPase has no autophosphorylation, dephosphorylation, ATPase and/or transport activity.

4. The method according to any one of claims , wherein the eukaryotic cells are mammalian cells.

5. The method according to any one of claims 1 to 5, wherein the labelled polyamine is a fluorescently labelled polyamine.

6. The method according to any one of claims 1 to 8, wherein the polyamine is putrescine, spermine or spermidine.

7. The method according to any one of claims 1 to 9, wherein the cell cultures are further subjected to oxidative stress.

8. A kit comprising:
a first eukaryotic (mammalian) cell line wherein the endogenous P5B type ATPase gene is inactivated, **characterised in that** the cell line comprises an inserted copy of a **wild type or disease causing mutant** P5B type ATPase, and
a second eukaryotic (mammalian) cell line wherein the endogenous P5B type ATPase gene is inactivated, **characterised in that** the cell line comprises an inserted copy of a an inactivated P5B type ATPase.

9. The kit according to claim 17, wherein the first and the second cell line have comparable expression levels of said inserted ATPase.

10. The kit according to claim 17 or 18, wherein the first and the second cell line have comparable endocytosis activity.

11. The kit according to any one of claims 17 to 19, wherein an inactivated P5B type ATPase is a catalytic inactive mutant lacking autophosphorylation, dephosphorylation, ATPase or transport activity.

12. The kit according to any one of claims 17 to 20, wherein the catalytic inactive mutant is the D508N mutant, with reference to the numbering of ATP13A2 variant 2.

13. An in vitro method of screening compounds modulating P5B type ATPase activity comprising the steps of:
a) providing a cell membrane fraction of cells expressing wild type P5B type ATPase or a disease causing mutant thereof in a buffered solution comprising:
- polyamine as transported substrate
- ATP
- a solubilising or membrane permeabilizing agent
- and a test compound
b) measuring ATP hydrolysis (by e.g. luminescence by measuring the released ADP, or colorimetric by measuring inorganic phosphate production)
c) comparing the measured ATP hydrolysis with the value of a control experiment without test compound, with vehicle control or with reference compounds that are modulators of P5B ATPase or polyamine transport activity.
d) wherein a difference in ATP hydrolysis is indicative of a test compound modulation P5B type ATPase activity .

14. An in vitro method of screening compounds modulating recombinant and purified P5B ATPase activity comprising the steps of:
a) Providing a buffered solution comprising solubilised purified P5B-ATPase such as ATP13A2
- a polyamine
- ATP
- and a regulatory lipid or agent that targets the auto-regulatory domain of the P5B-ATPase
b) adding a test compound,
c) determining ATPase activity, and comparing the determined active with the activity of a control experiment without test compound, with vehicle control or with reference compounds that are modulators of P5B ATPase or polyamine transport activity.
